# EUROPEAN PATENT APPLICATION

(11) **EP 4 273 148 A1**
(43) Date of publication of application: **08.11.2023**
(21) Application number: 21915809.4
(22) Date of filing: 29.12.2021
(51) Int. Cl.: C07D 487/04, H01L 51/00, H01L 51/50

(54) **COMPOUND FOR ORGANIC OPTOELECTRONIC ELEMENT, COMPOSITION FOR ORGANIC OPTOELECTRONIC ELEMENT, ORGANIC OPTOELECTRONIC ELEMENT, AND DISPLAY DEVICE**

(30) Priority: 30.12.2020 KR 20200187970
(71) Applicant: Samsung SDI Co., Ltd., Yongin-si, Gyeonggi-do 17084 (KR)
(72) Inventor: LIM, Youngmook, Suwon-si, Gyeonggi-do 16678 (KR); KIM, Hyung Sun, Suwon-si, Gyeonggi-do 16678 (KR); JUNG, Ho Kuk, Suwon-si, Gyeonggi-do 16678 (KR); LEE, Mijin, Suwon-si, Gyeonggi-do 16678 (KR); JO, Youngkyoung, Suwon-si, Gyeonggi-do 16678 (KR)
(74) Representative: Michalski Hüttermann & Partner Patentanwälte mbB
(86) International application number: PCT/KR2021/020118
(87) International publication number: WO 2022/146011

(57) **Abstract**

Provided are a compound for an organic optoelectronic device represented by a combination of Chemical Formula 1 and Chemical Formula 2, a composition for an organic optoelectronic device including the same, an organic optoelectronic device, and a display device.

Details for Chemical Formula 1 and Chemical Formula 2 are as defined in the specification.

## Description

### [Technical Field]

A compound for an organic optoelectronic device, a composition for an organic optoelectronic device, an organic optoelectronic device, and a display device are disclosed.

### [Background Art]

An organic optoelectronic device (organic optoelectronic diode) is a device capable of converting electrical energy and optical energy to each other.

Organic optoelectronic devices may be largely divided into two types according to a principle of operation. One is a photoelectric device that generates electrical energy by separating excitons formed by light energy into electrons and holes, and transferring the electrons and holes to different electrodes, respectively and the other is light emitting device that generates light energy from electrical energy by supplying voltage or current to the electrodes.

Examples of the organic optoelectronic device include an organic photoelectric device, an organic light emitting diode, an organic solar cell, and an organic photoconductor drum.

Among them, organic light emitting diodes (OLEDs) are attracting much attention in recent years due to increasing demands for flat panel display devices. The organic light emitting diode is a device that converts electrical energy into light, and the performance of the organic light emitting diode is greatly influenced by an organic material between electrodes.

### [Disclosure]

### [Technical Problem]

An example embodiment provides a compound for an organic optoelectronic device having high efficiency and a long life-span.

Another example embodiment provides a composition for an organic optoelectronic device including the compound for an organic optoelectronic device.

Another example embodiment provides an organic optoelectronic device including the compound for an organic optoelectronic device or the composition for an organic optoelectronic device.

Another example embodiment provides a display device including the organic optoelectronic device.

### [Technical Solution]

According to an example embodiment, a compound for an organic optoelectronic device represented by a combination of Chemical Formula 1 and Chemical Formula 2 is provided.

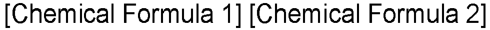

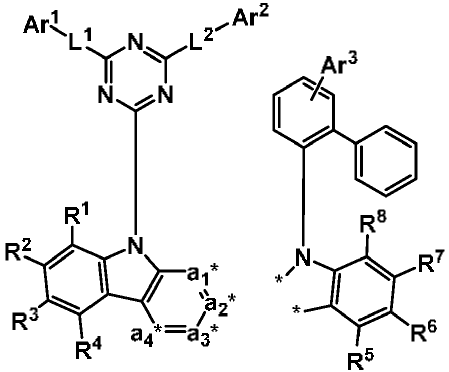

In Chemical Formula 1 and Chemical Formula 2,
adjacent two of a₁* to a₄* of Chemical Formula 1 are each independently linking carbon linked to * in Chemical Formula 2,
the rest two of a₁* to a₄* of Chemical Formula 1 not linked to * in Chemical Formula 2 are each independently C-L^{a}-R^{a},
Ar¹ and Ar² are each independently a substituted or unsubstituted C6 to C30 aryl group or a substituted or unsubstituted C2 to C30 heterocyclic group,
Ar³ is a substituted or unsubstituted C6 to C20 aryl group,
L^{a}, L¹, and L² are each independently a single bond, a substituted or unsubstituted C6 to C30 arylene group or a substituted or unsubstituted C2 to C30 heterocyclic group, and
R^{a}, and R¹ to R⁸ are each independently hydrogen, deuterium, a halogen, a substituted or unsubstituted C1 to C30 alkyl group, or a substituted or unsubstituted C6 to C30 aryl group.

According to another embodiment, a composition for an organic optoelectronic device includes a first compound, and a second compound.

The first compound is the same as described above, and the second compound may be a compound for an organic optoelectronic device represented by Chemical Formula 3; or a compound for an organic optoelectronic device represented by a combination of Chemical Formula 4 and Chemical Formula 5. 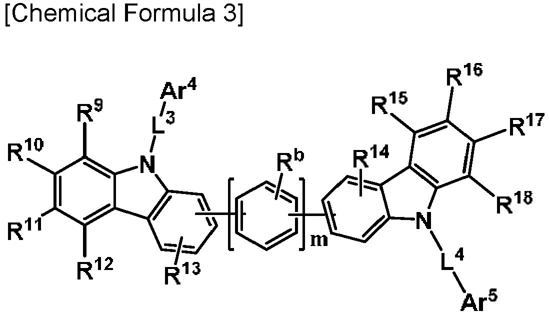 In Chemical Formula 3,
Ar⁴ and Ar⁵ are each independently a substituted or unsubstituted C6 to C20 aryl group or a substituted or unsubstituted C2 to C30 heterocyclic group,
L³ and L⁴ are each independently a single bond or a substituted or unsubstituted C6 to C20 arylene group,
R^{b} and R⁹ to R¹⁸ are each independently hydrogen, deuterium, a cyano group, a halogen, a substituted or unsubstituted amino group, a substituted or unsubstituted C1 to C30 alkyl group, a substituted or unsubstituted C6 to C30 aryl group, or a substituted or unsubstituted C2 to C30 heterocyclic group, and
m is an integer of 0 to 2;

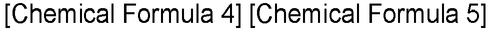

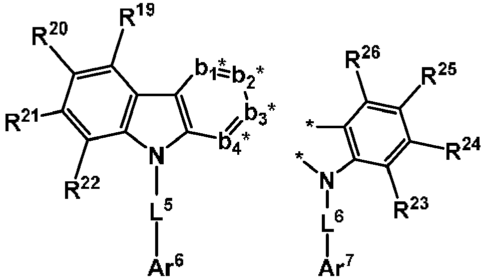

In Chemical Formulas 4 and 5,
Ar⁶ and Ar⁷ are each independently a substituted or unsubstituted C6 to C20 aryl group or a substituted or unsubstituted C2 to C30 heterocyclic group,
adjacent two of b₁* to b₄* of Chemical Formula 4 are each independently linking carbon linked to * in Chemical Formula 5,
the rest two of b₁* to b₄* of Chemical Formula 4 not linked to * in Chemical Formula 5 are each independently C-L^{b}-R^{c},
L^{b}, L⁵, and L⁶ are each independently a single bond or a substituted or unsubstituted C6 to C20 arylene group,
R^{c} and R¹⁹ to R²⁶ are each independently hydrogen, deuterium, a cyano group, a halogen, a substituted or unsubstituted amino group, a substituted or unsubstituted C1 to C30 alkyl group, a substituted or unsubstituted C6 to C30 aryl group, or a substituted or unsubstituted C2 to C30 heterocyclic group.

According to another embodiment, an organic optoelectronic device includes an anode and a cathode facing each other, and at least one organic layer between the anode and the cathode, wherein the organic layer includes the compound for an organic optoelectronic device or a composition for an organic optoelectronic device.

According to another embodiment, a display device including the organic optoelectronic device is provided.

### [Advantageous Effects]

An organic optoelectronic device having high efficiency and a long life-span may be realized.

### [Description of the Drawings]

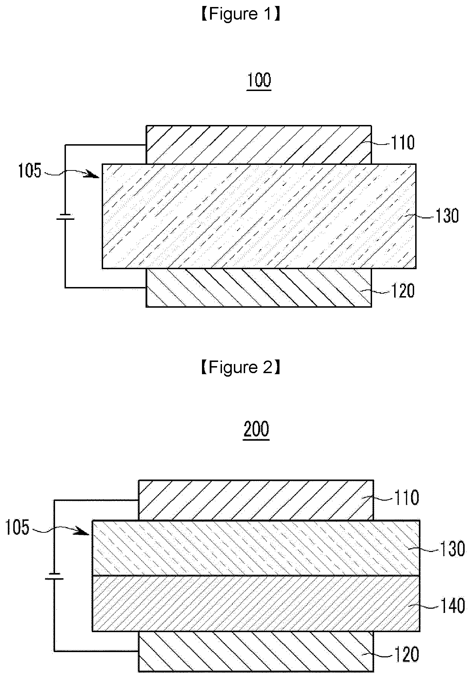

FIGS. 1 to 4 are cross-sectional views each illustrating an organic light emitting diode according to embodiments.

### <Description of symbols>

- 100, 200, 300, 400:: organic light emitting diode
- 105:: organic layer
- 110:: cathode
- 120:: anode
- 130:: light emitting layer
- 140:: hole transport region
- 150:: electron transport region

### [Best Mode]

Hereinafter, embodiments of the present invention are described in detail. However, these embodiments are exemplary, the present invention is not limited thereto and the present invention is defined by the scope of claims.

In the present specification, when a definition is not otherwise provided, "substituted" refers to replacement of at least one hydrogen of a substituent or a compound by deuterium, a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 trifluoroalkyl group, a cyano group, or a combination thereof.

In one example of the present invention, the "substituted" refers to replacement of at least one hydrogen of a substituent or a compound by deuterium, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, or a cyano group. In specific example of the present invention, the "substituted" refers to replacement of at least one hydrogen of a substituent or a compound by deuterium, a C1 to C20 alkyl group, a C6 to C30 aryl group, or a cyano group. In specific example of the present invention, the "substituted" refers to replacement of at least one hydrogen of a substituent or a compound by deuterium, a C1 to C5 alkyl group, a C6 to C18 aryl group, or a cyano group. In specific example of the present invention, the "substituted" refers to replacement of at least one hydrogen of a substituent or a compound by deuterium, a cyano group, a methyl group, an ethyl group, a propyl group, a butyl group, a phenyl group, a biphenyl group, a terphenyl group, or a naphthyl group.

In the present specification, when a definition is not otherwise provided, "hetero" refers to one including one to three heteroatoms selected from N, O, S, P, and Si, and remaining carbons in one functional group.

In the present specification, "an aryl group" refers to a group including at least one hydrocarbon aromatic moiety, and all elements of the hydrocarbon aromatic moiety have p-orbitals which form conjugation, for example a phenyl group, a naphthyl group, and the like, two or more hydrocarbon aromatic moieties may be linked by a sigma bond and may be, for example a biphenyl group, a terphenyl group, a quarterphenyl group, and the like, and two or more hydrocarbon aromatic moieties are fused directly or indirectly to provide a nonaromatic fused ring, for example a fluorenyl group.

The aryl group may include a monocyclic, polycyclic, or fused ring polycyclic (i.e., rings sharing adjacent pairs of carbon atoms) functional group.

In the present specification, "a heterocyclic group" is a generic concept of a heteroaryl group, and may include at least one heteroatom selected from N, O, S, P, and Si instead of carbon (C) in a cyclic compound such as an aryl group, a cycloalkyl group, a fused ring thereof, or a combination thereof. When the heterocyclic group is a fused ring, the entire ring or each ring of the heterocyclic group may include one or more heteroatoms.

For example, "a heteroaryl group" may refer to an aryl group including at least one heteroatom selected from N, O, S, P, and Si. Two or more heteroaryl groups are linked by a sigma bond directly, or when the heteroaryl group includes two or more rings, the two or more rings may be fused. When the heteroaryl group is a fused ring, each ring may include one to three heteroatoms.

More specifically, the substituted or unsubstituted C6 to C30 aryl group may be a substituted or unsubstituted phenyl group, a substituted or unsubstituted naphthyl group, a substituted or unsubstituted anthracenyl group, a substituted or unsubstituted phenanthrenyl group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted o-terphenyl group, a substituted or unsubstituted chrysenyl group, a substituted or unsubstituted triphenylene group, a substituted or unsubstituted perylenyl group, a substituted or unsubstituted fluorenyl group, a substituted or unsubstituted indenyl group, a substituted or unsubstituted furanyl group, or a combination thereof, but is not limited thereto.

More specifically, the substituted or unsubstituted C2 to C30 heterocyclic group may be a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted arcridinyl group, a substituted or unsubstituted phenazinyl group, a substituted or unsubstituted phenothiazinyl group, a substituted or unsubstituted phenoxazinyl group, a substituted or unsubstituted carbazolyl group, a substituted or unsubstituted dibenzofuranyl group, or a substituted or unsubstituted dibenzothiophenyl group, or a combination thereof, but is not limited thereto.

In the present specification, hole characteristics refer to an ability to donate an electron to form a hole when an electric field is applied and that a hole formed in the anode may be easily injected into the light emitting layer and transported in the light emitting layer due to conductive characteristics according to a highest occupied molecular orbital (HOMO) level.

In addition, electron characteristics refer to an ability to accept an electron when an electric field is applied and that electron formed in the cathode may be easily injected into the light emitting layer and transported in the light emitting layer due to conductive characteristics according to a lowest unoccupied molecular orbital (LUMO) level.

Hereinafter, a compound for an organic optoelectronic device according to an example embodiment is described.

A compound for an organic optoelectronic device according to an example embodiment is represented by a combination of Chemical Formula 1 and Chemical Formula 2.

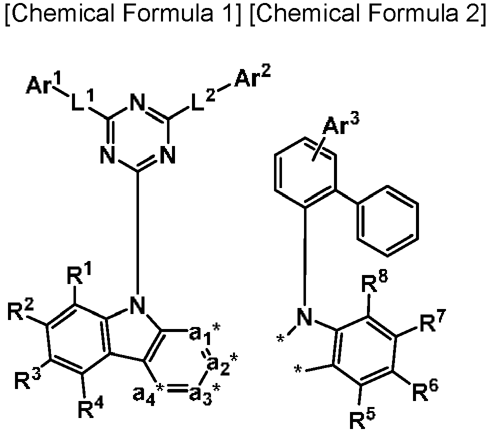

In Chemical Formula 1 and Chemical Formula 2,
adjacent two of a₁* to a₄* of Chemical Formula 1 are each independently linking carbon linked to * in Chemical Formula 2,
the rest two of a₁* to a₄* of Chemical Formula 1 not linked to * in Chemical Formula 2 are each independently C-L^{a}-R^{a},
Ar¹ and Ar² are each independently a substituted or unsubstituted C6 to C30 aryl group or a substituted or unsubstituted C2 to C30 heterocyclic group,
Ar³ is a substituted or unsubstituted C6 to C20 aryl group,
L^{a}, L¹, and L² are each independently a single bond, a substituted or unsubstituted C6 to C30 arylene group or a substituted or unsubstituted C2 to C30 heterocyclic group, and
R^{a}, and R¹ to R⁸ are each independently hydrogen, deuterium, a halogen, a substituted or unsubstituted C1 to C30 alkyl group, or a substituted or unsubstituted C6 to C30 aryl group.

In the compound represented by the combination of Chemical Formula 1 and Chemical Formula 2, one of the substituents in the N direction of the indolocarbazole core is a substituted or unsubstituted triazine group, and the other is a phenylene moiety having a phenyl group in the ortho direction and an additional substituent in the other direction.

In this structure, the triazine and the ortho-phenylene moiety including the additional substituent in the indolocarbazole skeleton in which the N-direction substituents are positioned in parallel have a steric structural hindrance to each other, thereby reducing a degree of freedom of the molecule and arranging the molecules in a certain direction. This increases intermolecular orientation to increase intermolecular electron and hole mobility, and relatively high efficiency and life-span may be exhibited.

The combination of Chemical Formula 1 and Chemical Formula 2 may be represented by one of Chemical Formula 1A to Chemical Formula 1F.

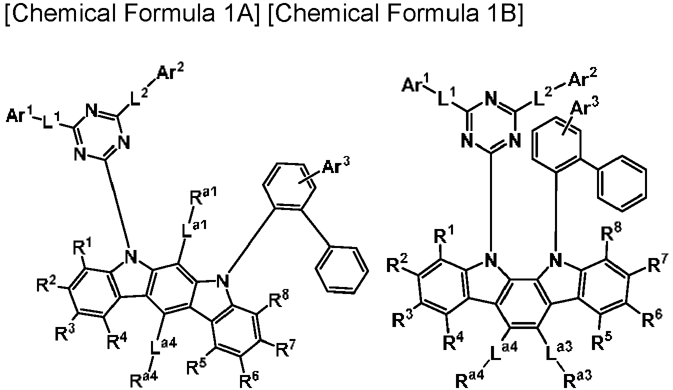

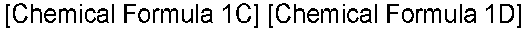

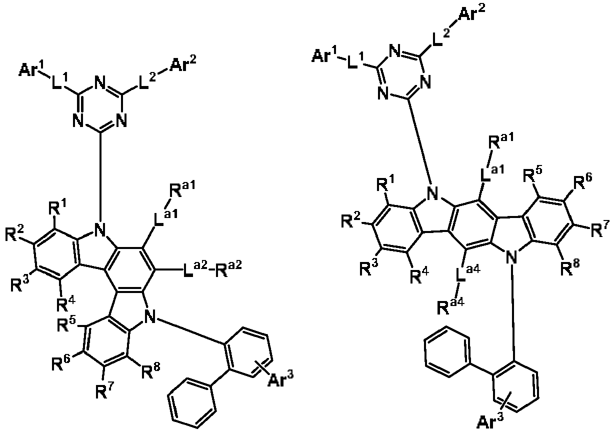

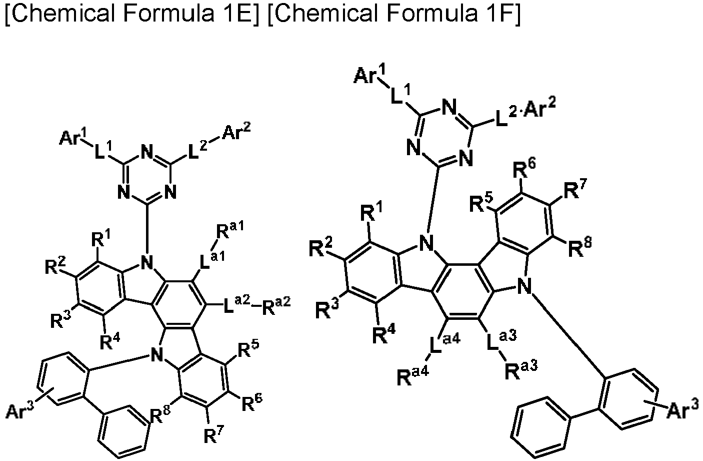

In Chemical Formula 1A to Chemical Formula 1F,
Ar¹ to Ar³, L¹, L², and R¹ to R⁸ are the same as described above,
L^{a1} to L^{a4} are the same as the definition of L^{a} described above, and
R^{a1} to R^{a4} are the same as the definition of R^{a} described above.

For example, the combination of Chemical Formula 1 and Chemical Formula 2 may be represented by Chemical Formula 1B.

As a specific example, Chemical Formula 1B may be represented by, for example, one of Chemical Formula 1B-1 to Chemical Formula 1B-4, depending on a substitution direction of the additional substituent included in the ortho-phenylene moiety in the indolocarbazole skeleton.

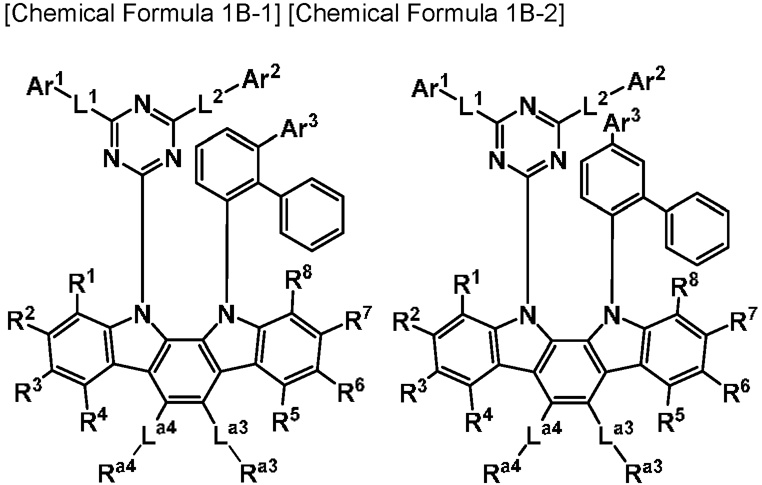

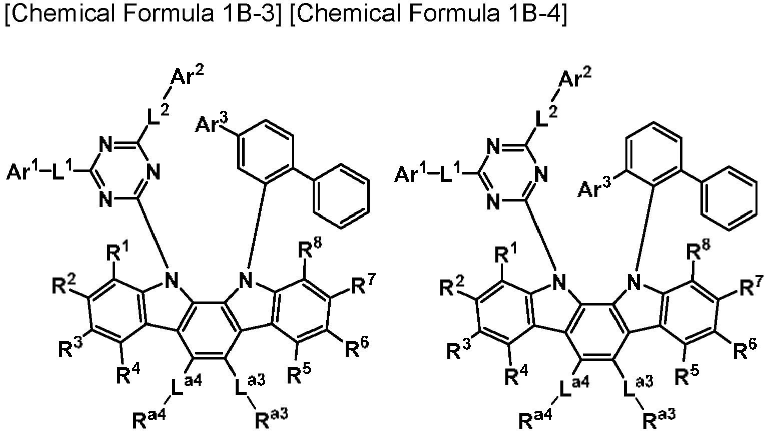

In Chemical Formula 1B-1 to Chemical Formula 1B-4,
Ar¹ to Ar³, L¹, L², L^{a3}, L^{a4}, and R¹ to R⁸ are the same as described above.

In an example embodiment, Ar³ may be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, or a substituted or unsubstituted triphenylene group.

In an example embodiment, Ar¹ and Ar² may each independently be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted triphenylene group, a substituted or unsubstituted fluorenyl group, a substituted or unsubstituted dibenzofuranyl group, a substituted or unsubstituted dibenzothiophenyl group, or a substituted or unsubstituted dibenzosilolyl group.

In a specific example embodiment, Ar¹ and Ar² may each independently be a substituted or unsubstituted phenyl group, a substituted or unsubstituted biphenyl group, a substituted or unsubstituted terphenyl group, a substituted or unsubstituted quaterphenyl group, or a substituted or unsubstituted naphthyl group.

In an example embodiment, L¹ and L² may each independently be a single bond, a substituted or unsubstituted phenylene group, a substituted or unsubstituted biphenylene group, a substituted or unsubstituted dibenzofuranylene group, or a substituted or unsubstituted dibenzothiophenylene group.

In a specific embodiment, L¹ to L³ may each independently be a single bond, or a substituted or unsubstituted phenylene group.

For example, *-L¹-Ar¹ and *-L²-Ar² may be each independently selected from substituents of Group I .

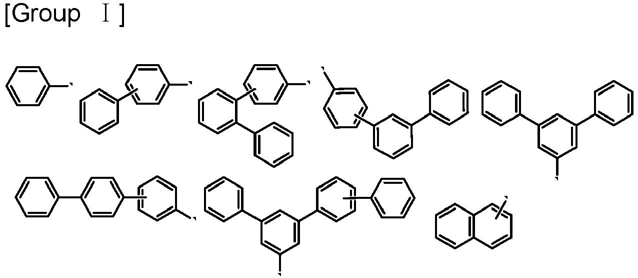

In Group I , * is a linking point.

When Ar¹ and Ar² are substituted, the substituent may be a C6 to C12 aryl group. More specifically, the substituent may be a phenyl group.

For example, at least one of Ar¹ and Ar² may be a substituted or unsubstituted C10 to C30 aryl group.

In a specific example, at least one of Ar¹ and Ar² may be a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted triphenylene group, or a substituted or unsubstituted fluorenyl group.

In an example embodiment, R¹ to R⁸ may each independently be hydrogen or a substituted or unsubstituted phenyl group.

In a specific embodiment, each of R¹ to R⁸ may be hydrogen.

The more specific example of the compound for an organic optoelectronic device represented by the combination of Chemical Formula 1 and Chemical Formula 2 may include the compounds of Group 1, but is not limited thereto.

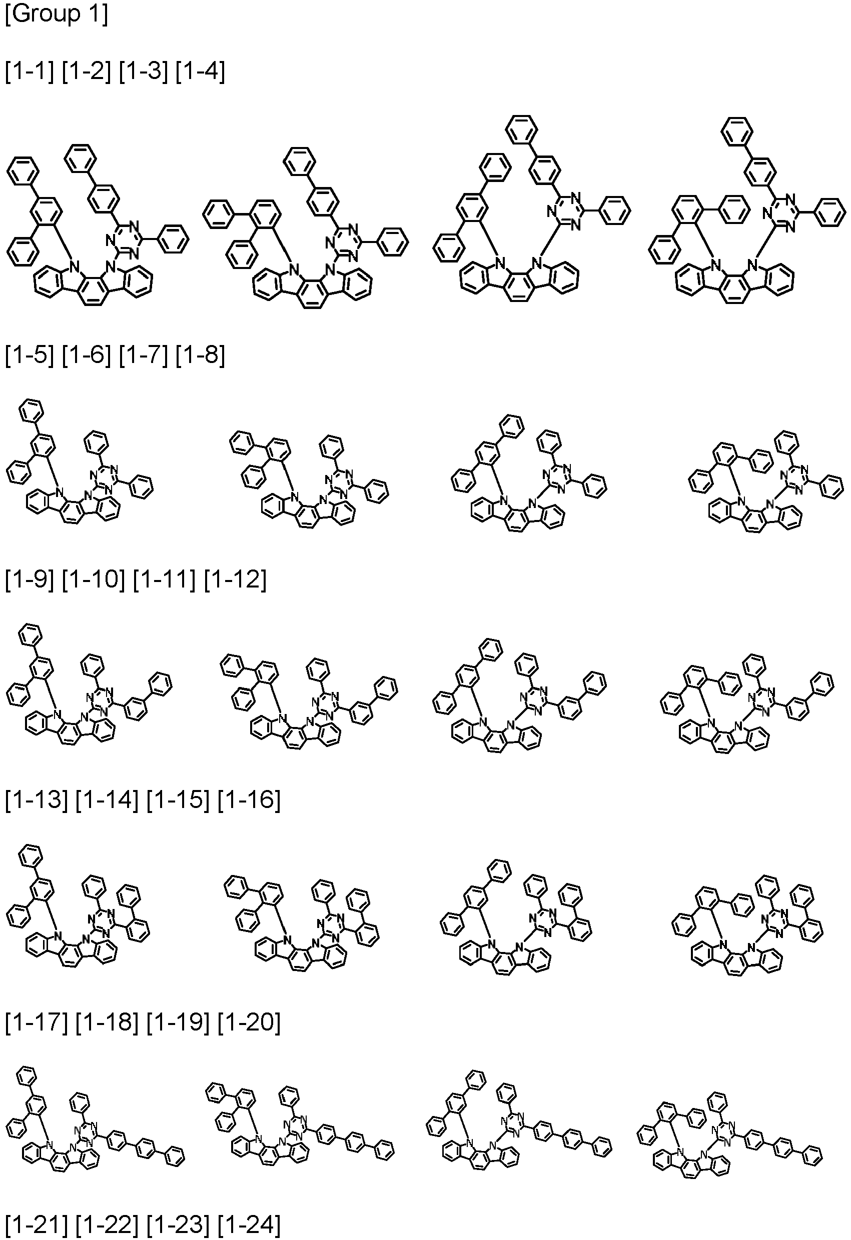

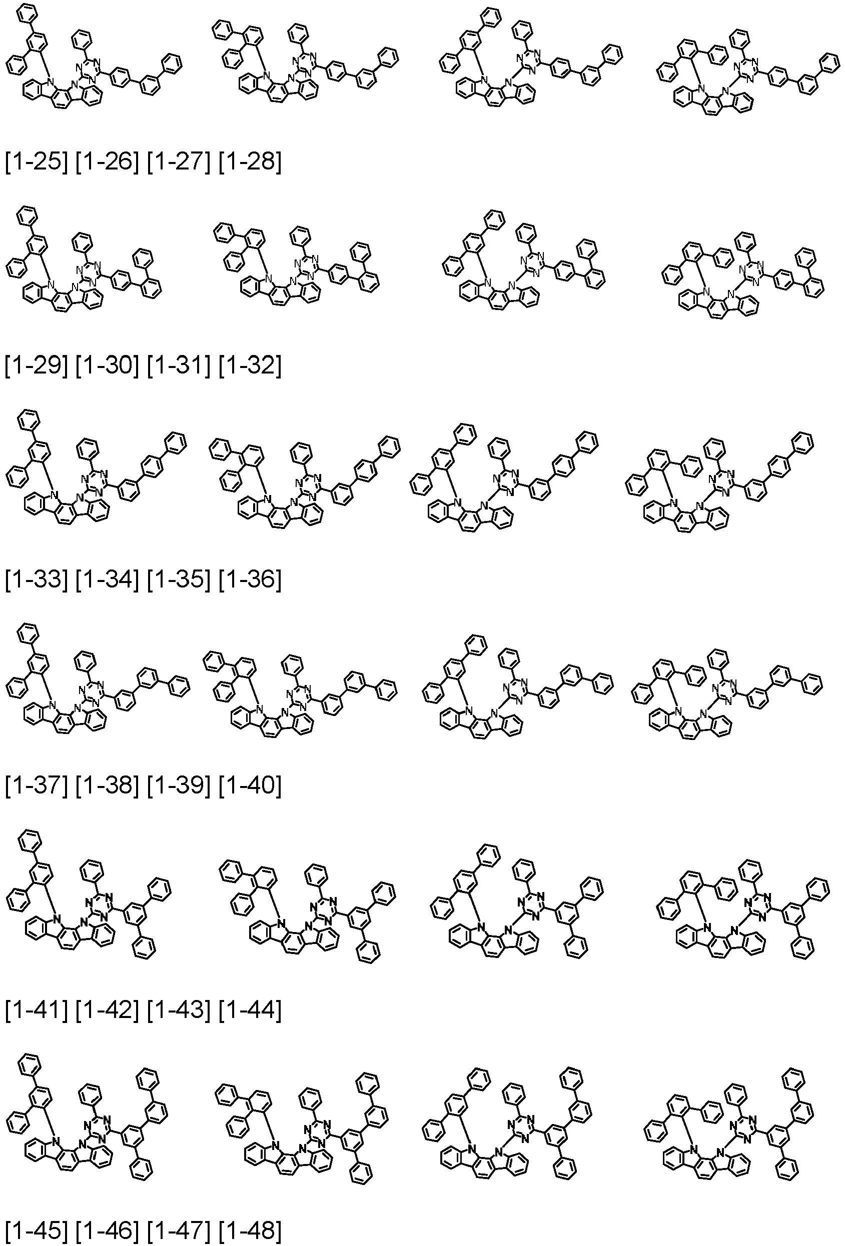

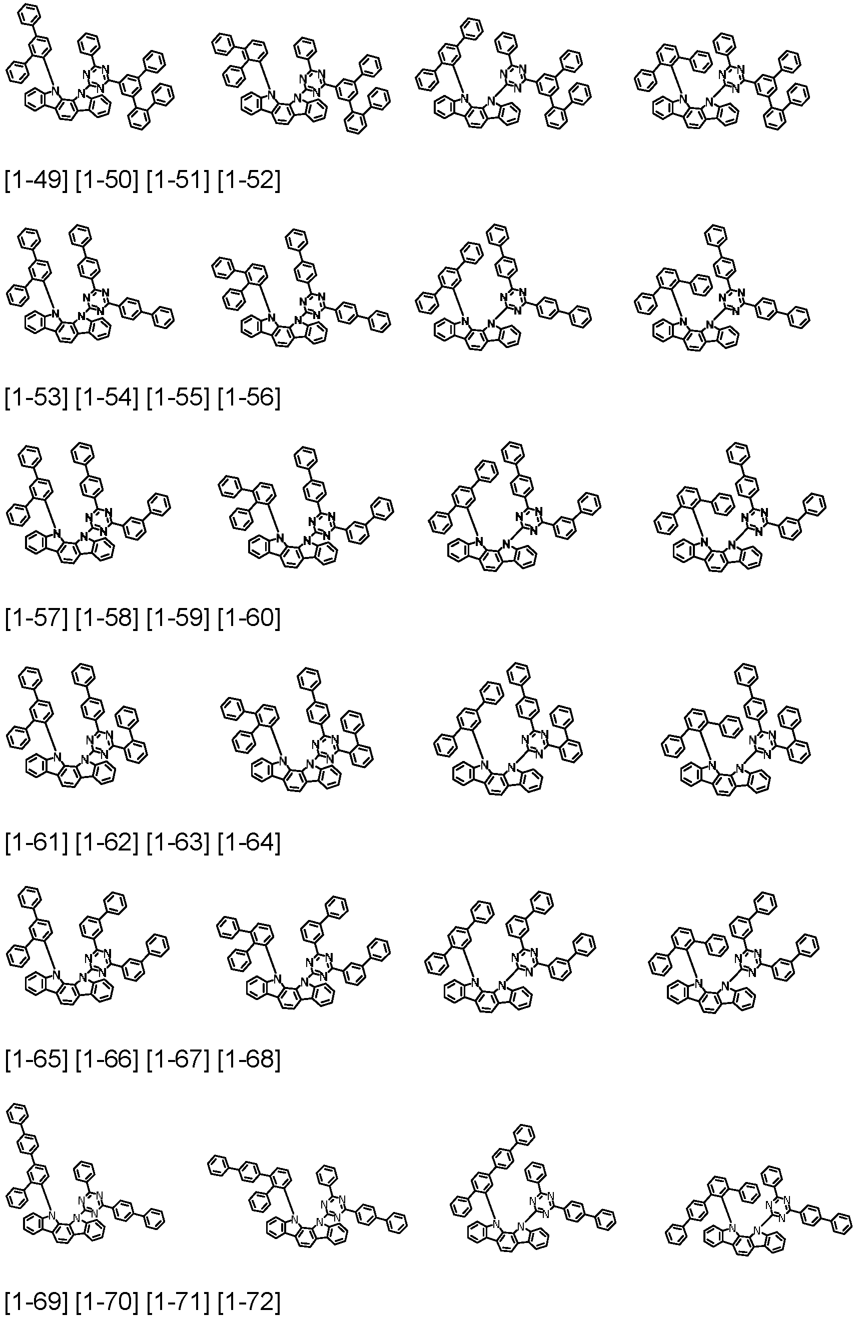

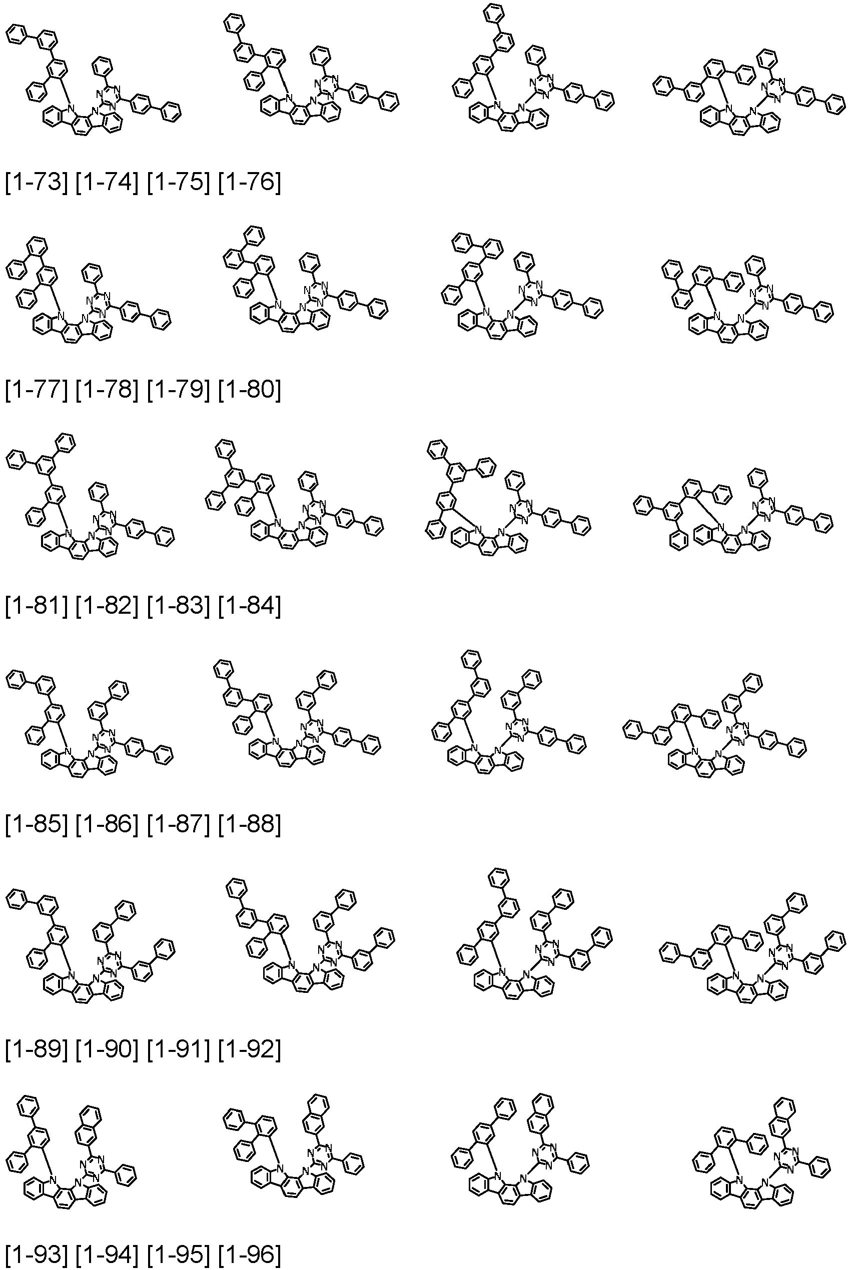

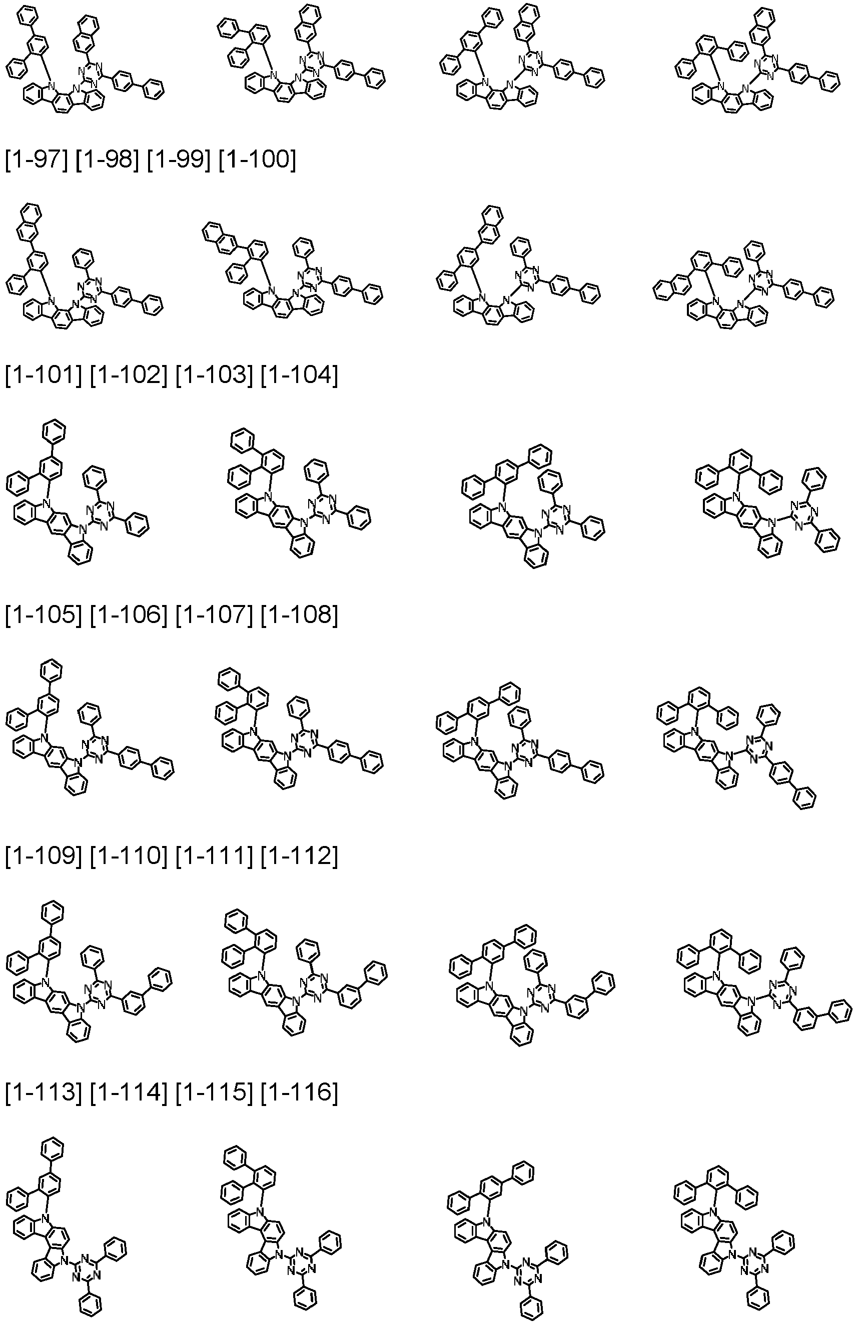

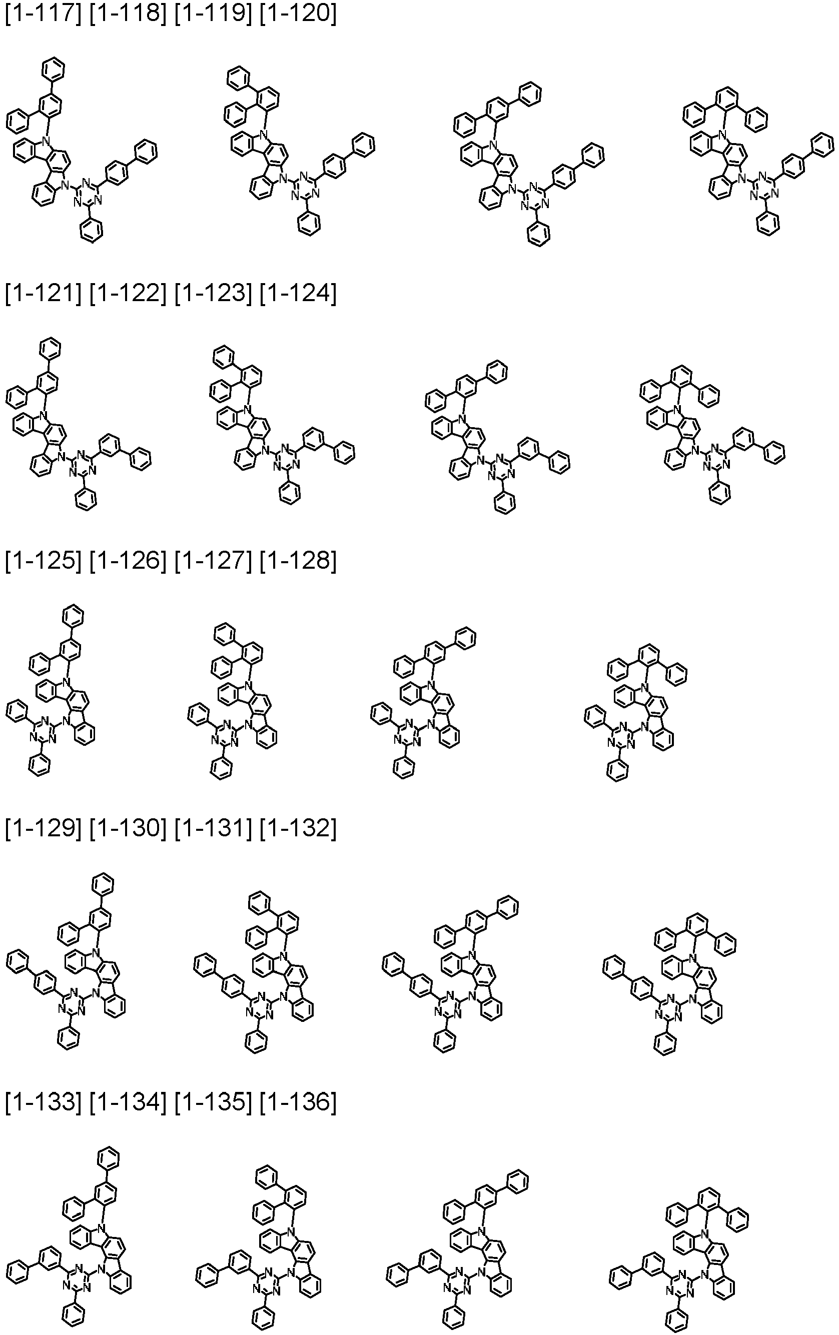

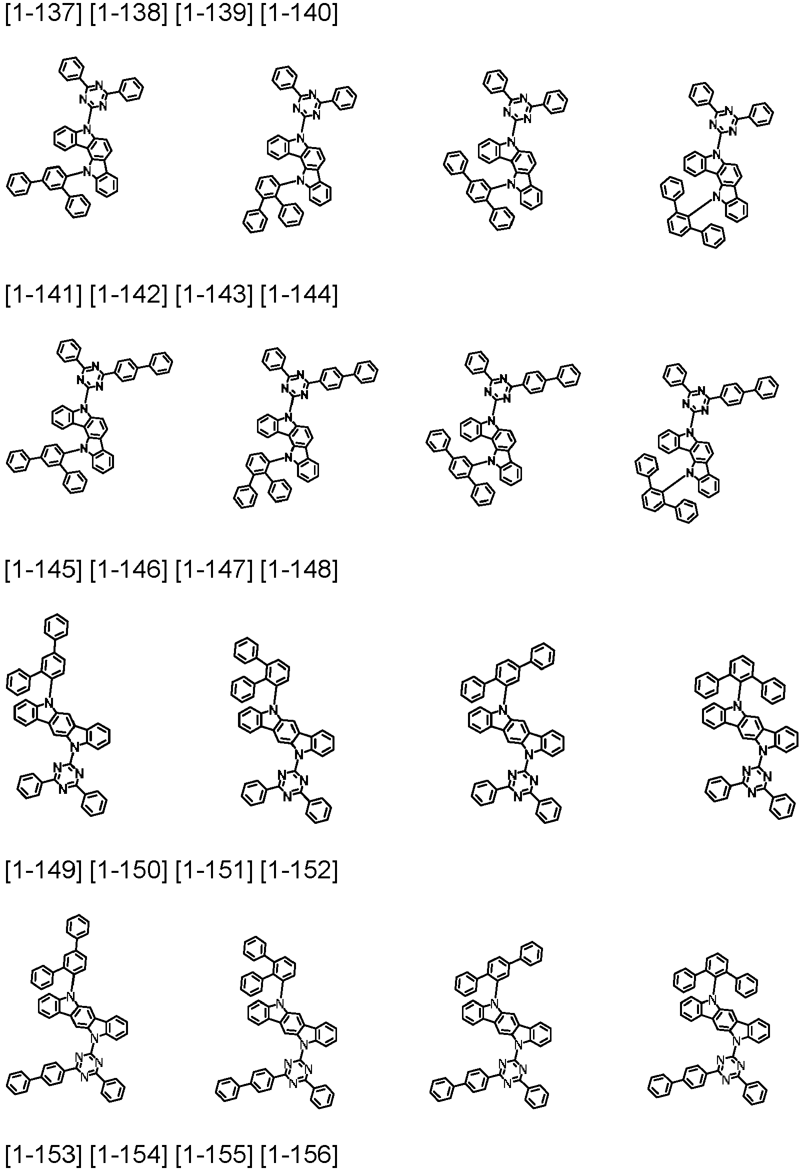

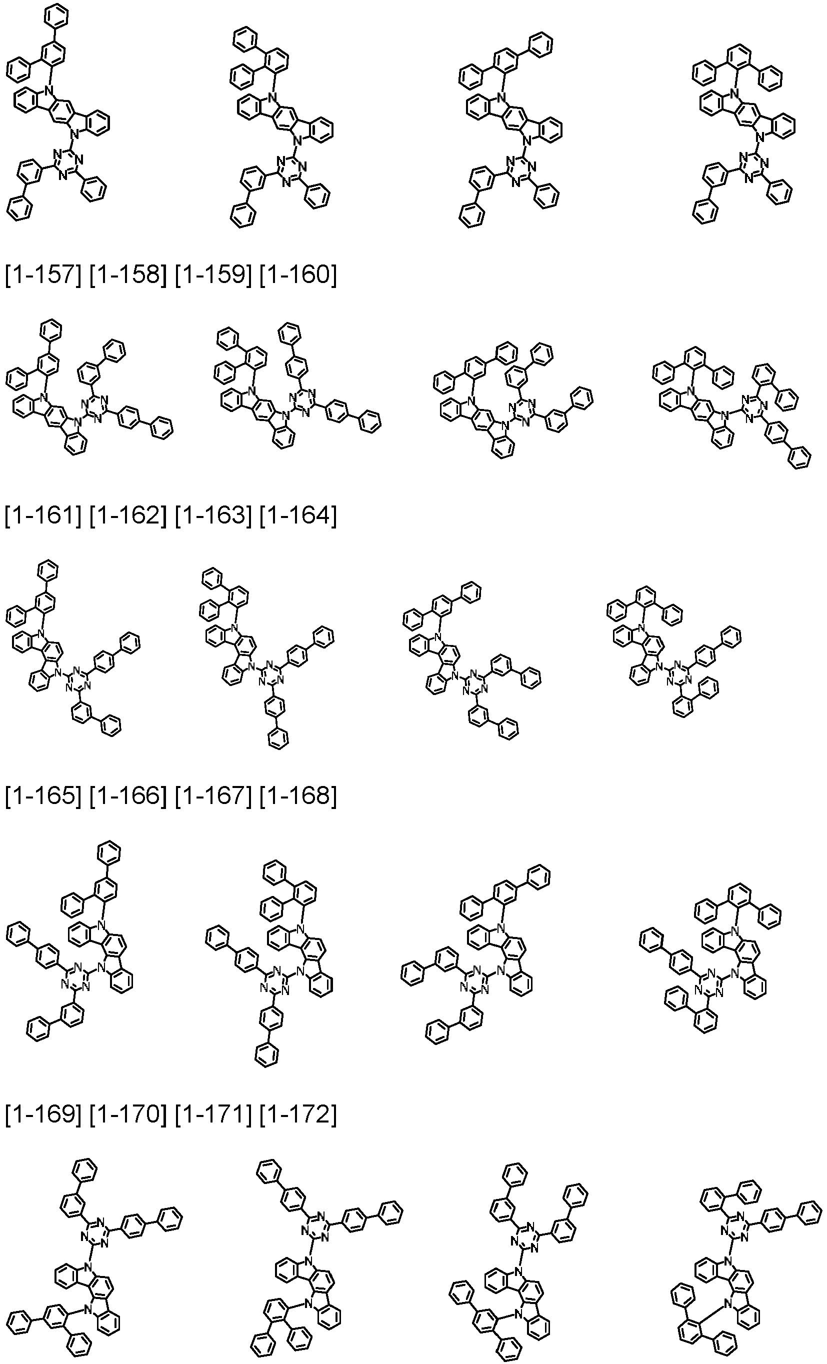

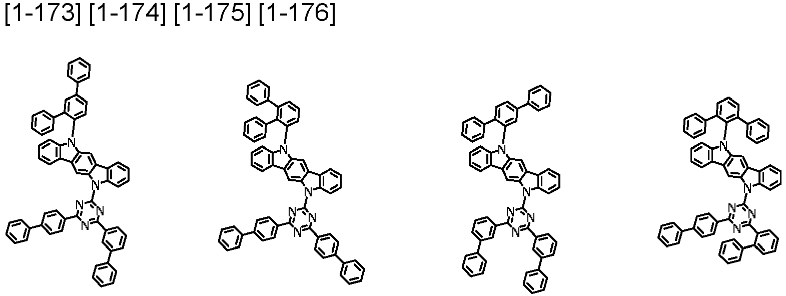

A composition for an organic optoelectronic device according to another embodiment includes a first compound, and a second compound, wherein the first compound may be the aforementioned compound for an organic optoelectronic device and the second compound may be a compound for an organic optoelectronic device represented by Chemical Formula 3; or a compound for an organic optoelectronic device represented by the combination of Chemical Formula 4 and Chemical Formula 5.

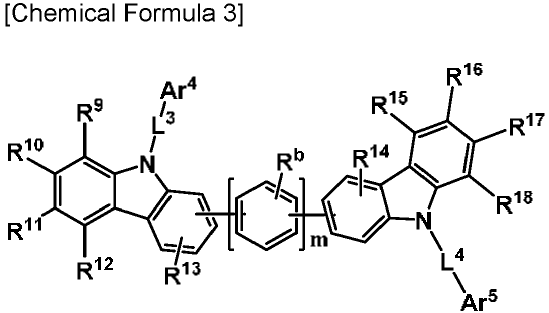

In Chemical Formula 3,
Ar⁴ and Ar⁵ are each independently a substituted or unsubstituted C6 to C20 aryl group or a substituted or unsubstituted C2 to C30 heterocyclic group,
L³ and L⁴ are each independently a single bond or a substituted or unsubstituted C6 to C20 arylene group,
R^{b} and R⁹ to R¹⁸ are each independently hydrogen, deuterium, a cyano group, a halogen, a substituted or unsubstituted amino group, a substituted or unsubstituted C1 to C30 alkyl group, a substituted or unsubstituted C6 to C30 aryl group, or a substituted or unsubstituted C2 to C30 heterocyclic group, and
m is an integer of 0 to 2;

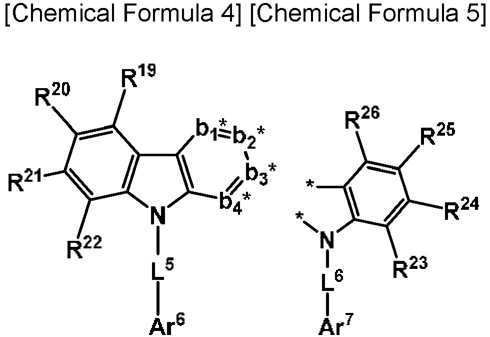

In Chemical Formulas 4 and 5,
Ar⁶ and Ar⁷ are each independently a substituted or unsubstituted C6 to C20 aryl group or a substituted or unsubstituted C2 to C30 heterocyclic group,
adjacent two of b₁* to b₄* of Chemical Formula 4 are each independently linking carbon linked to * in Chemical Formula 5,
the rest two of b₁* to b₄* of Chemical Formula 4 not linked to * in Chemical Formula 5 are each independently C-L^{b}-R^{c},
L^{b}, L⁵, and L⁶ are each independently a single bond or a substituted or unsubstituted C6 to C20 arylene group, and
R^{c} and R¹⁹ to R²⁶ are each independently hydrogen, deuterium, a cyano group, a halogen, a substituted or unsubstituted amino group, a substituted or unsubstituted C1 to C30 alkyl group, a substituted or unsubstituted C6 to C30 aryl group, or a substituted or unsubstituted C2 to C30 heterocyclic group.

The second compound may be used in the light emitting layer together with the first compound to increase mobility of charges and improve stability, thereby improving luminous efficiency and life-span characteristics.

For example, Ar⁴ and Ar⁵ of Chemical Formula 3 may each independently be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted triphenylenyl group, a substituted or unsubstituted carbazolyl group, a substituted or unsubstituted dibenzothiophenyl group, a substituted or unsubstituted dibenzofuranyl group, or a substituted or unsubstituted fluorenyl group,
L³ and L⁴ of Chemical Formula 3 may each independently be a single bond, a substituted or unsubstituted phenylene group, or a substituted or unsubstituted biphenylene group,
R^{b} and R⁹ to R¹⁸ of Chemical Formula 3 may each independently be hydrogen, deuterium, or a substituted or unsubstituted C6 to C12 aryl group,
m may be 0 or 1.

"Substituted" of Chemical Formula 3 refers to replacement of at least one hydrogen by deuterium, a C1 to C4 alkyl group, a C6 to C18 aryl group, or a C2 to C30 heteroaryl group.

In a specific embodiment of the present invention, Chemical Formula 3 may be represented by one of Chemical Formula 3-1 to Chemical Formula 3-15.

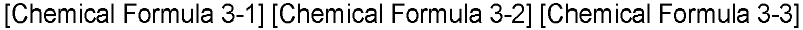

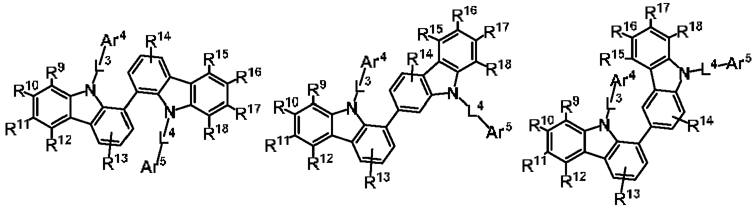

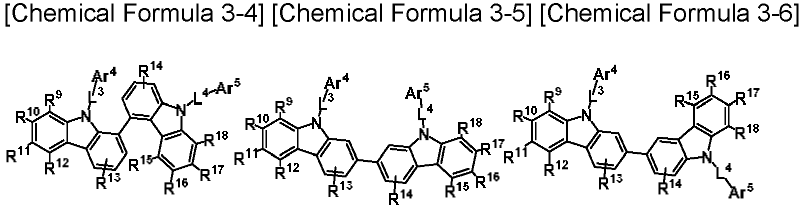

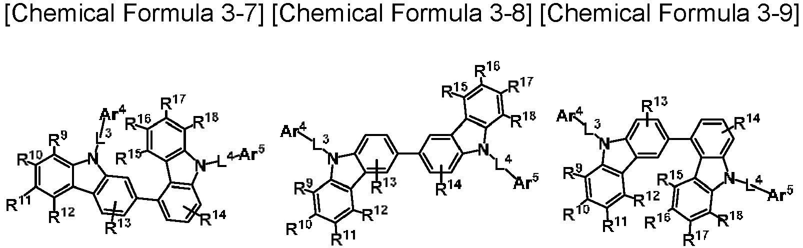

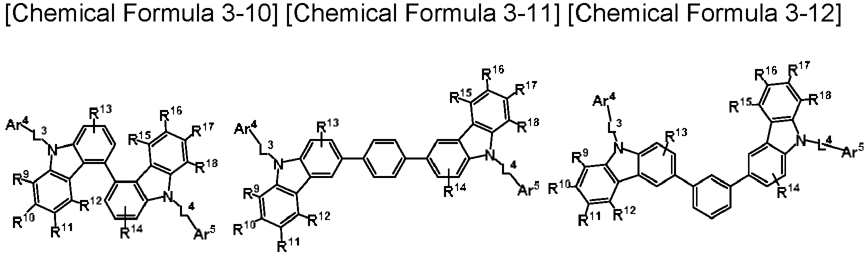

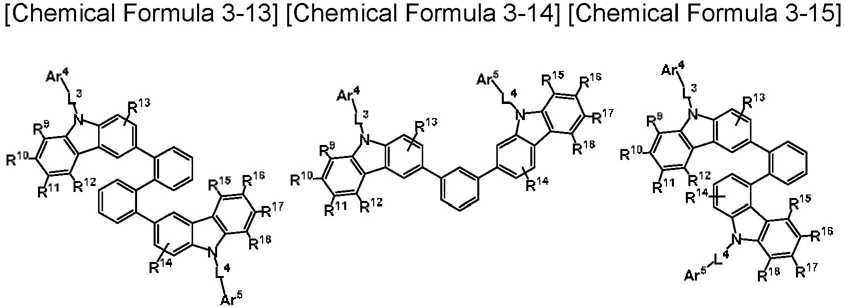

In Chemical Formula 3-1 to Chemical Formula 3-15, R⁹ to R¹⁸ may each independently be hydrogen or a substituted or unsubstituted C6 to C12 aryl group, and *-L³-Ar⁴ and *-L⁴-Ar⁵ may each independently be one of substituents of Group II.

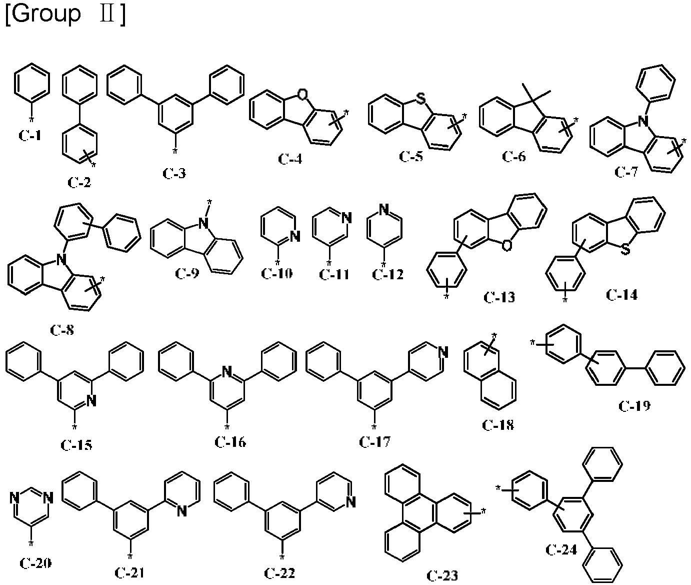

In Group II, * is a linking point.

In an example embodiment, Chemical Formula 3 may be represented by Chemical Formula 3-8.

In addition, *-L³-Ar⁴ and *-L⁴-Ar⁵ of Chemical Formula 3-8 may each independently be selected from Group II, and may be, for example, one of C-1, C-2, C-3, C-4, C -7, C-8, and C-9.

For example, the second compound represented by the combination of Chemical Formula 4 and Chemical Formula 5 may be represented by one of Chemical Formula Chemical Formula 4A, Chemical Formula 4B, Chemical Formula 4C, Chemical Formula 4D and Chemical Formula 4E.

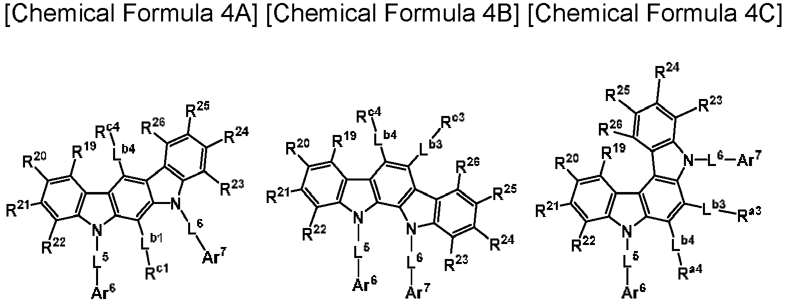

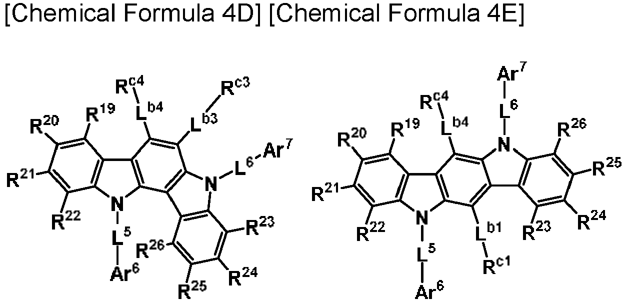

In Chemical Formula 4A to Chemical Formula 4E, Ar⁶, Ar⁷, L⁵, L⁶, and R¹⁹ to R²⁶ are the same as described above,
L^{b1} to L^{b4} are the same as the definitions of L⁵ and L⁶ described above, and
R^{c1} to R^{c4} have the same definitions as R¹⁹ to R²⁶ described above.

For example, Ar⁶ and Ar⁷ of Chemical Formulas 4 and 5 may each independently be a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group, and

R^{c1} to R^{c4} and R¹⁹ to R²⁶ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

In a specific embodiment of the present invention, Ar⁶ and Ar⁷ in Chemical Formulas 4 and 5 may be each independently selected from the substituents of Group II.

In an example embodiment, R^{c1} to R^{c4} and R¹⁹ to R²⁶ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

For example, R^{c1} to R^{c4} and R¹⁹ to R²⁶ may each independently be hydrogen, deuterium, a cyano group, or a substituted or unsubstituted phenyl group, and
in a specific example embodiment, R^{c1} to R^{c4}, and R¹⁹ to R²⁶ may each independently be hydrogen, or a phenyl group.

In a specific example embodiment, the second compound may be represented by Chemical Formula 3-8, wherein Ar⁴ and Ar⁶ of Chemical Formula 3-8 may each independently be a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group, L³ and L⁴ may each independently be a single bond, or a substituted or unsubstituted C6 to C20 arylene group, R⁹ to R¹⁸ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

In another specific example embodiment, the second compound may be represented by Chemical Formula 4C, wherein L^{b1} to L^{b4} of Chemical Formula 4C may be a single bond, L⁵ and L⁶ may each independently be a single bond or a substituted or unsubstituted C6 to C12 arylene group, R¹⁹ to R²⁶, R^{c1} to R^{c4} may each independently be hydrogen or a phenyl group, and Ar⁶ and Ar⁷ may each independently be a substituted or unsubstituted phenyl group, a substituted or unsubstituted biphenyl group, or a substituted or unsubstituted terphenyl group.

For example, the second compound may be one selected from compounds of Group 2, but is not limited thereto.

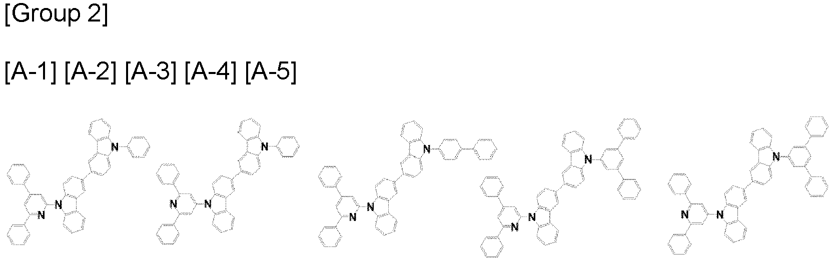

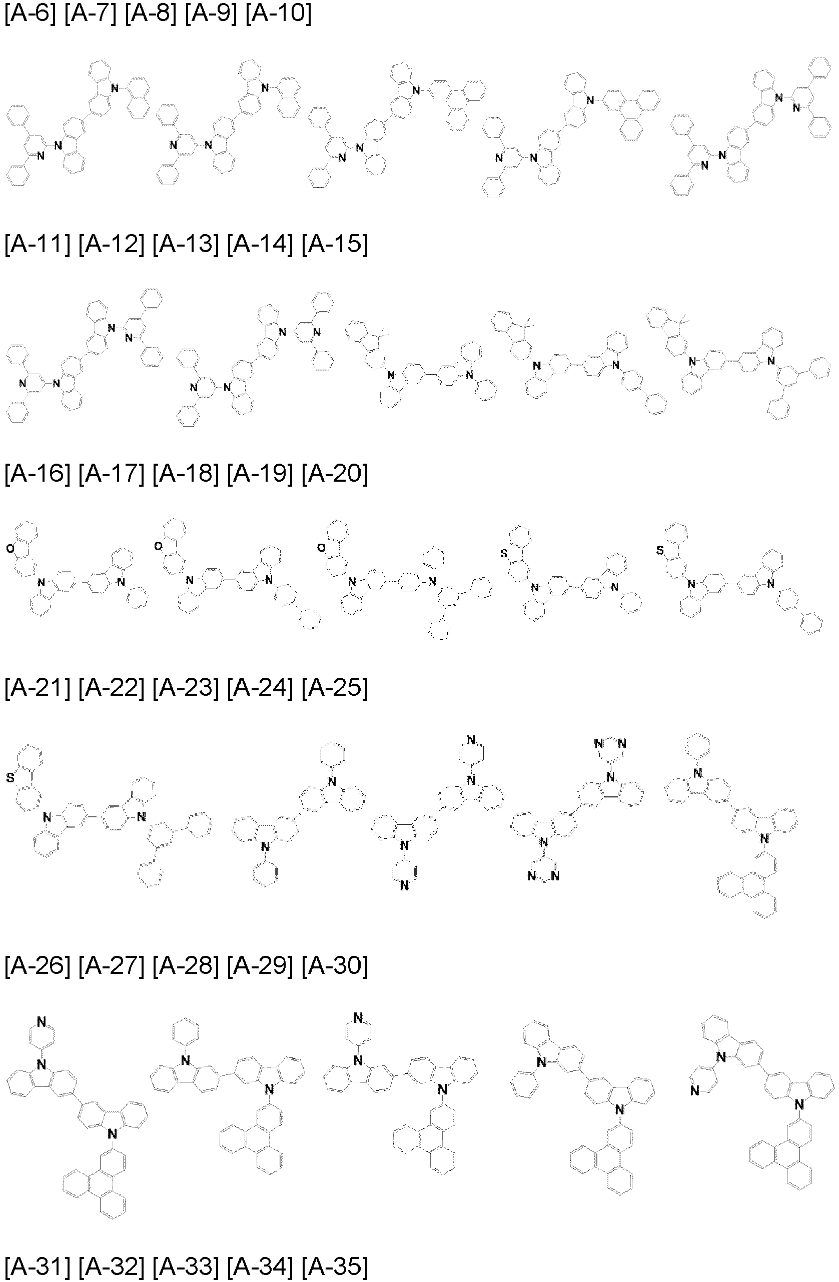

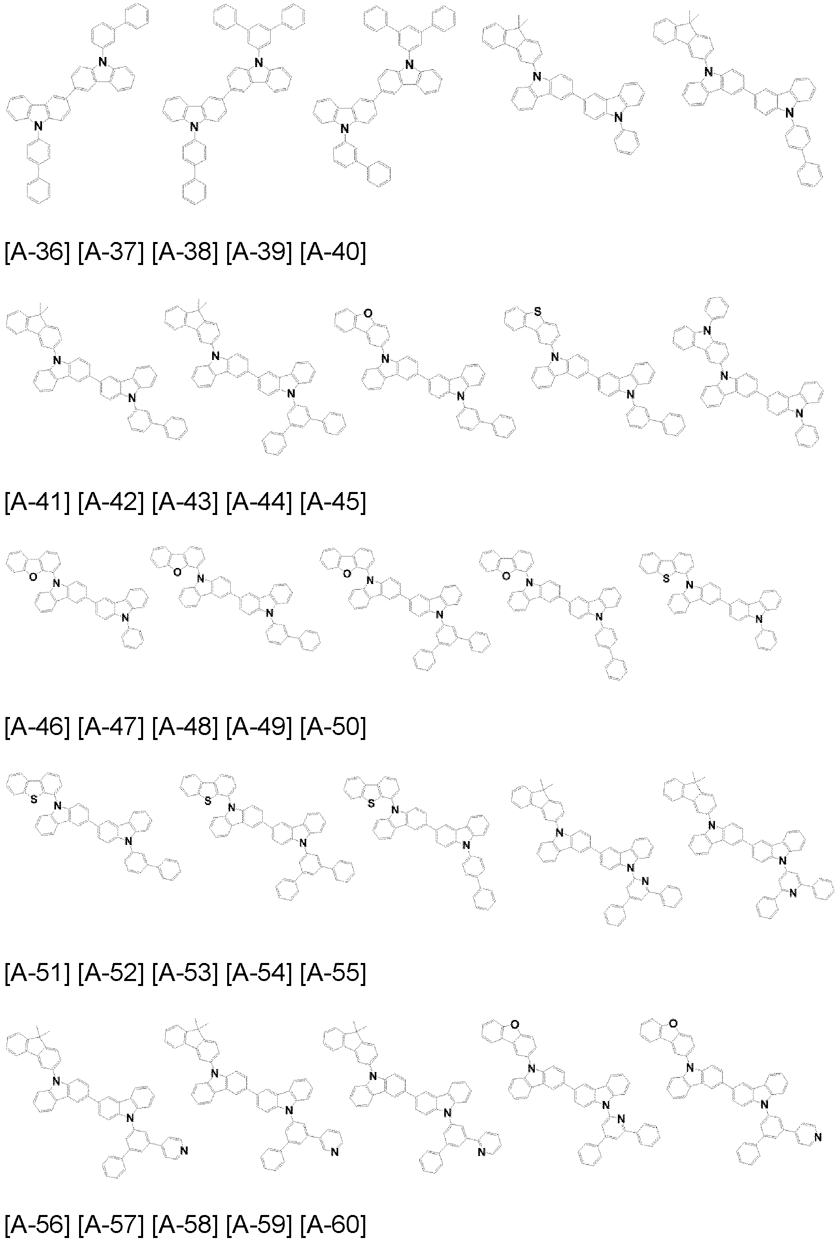

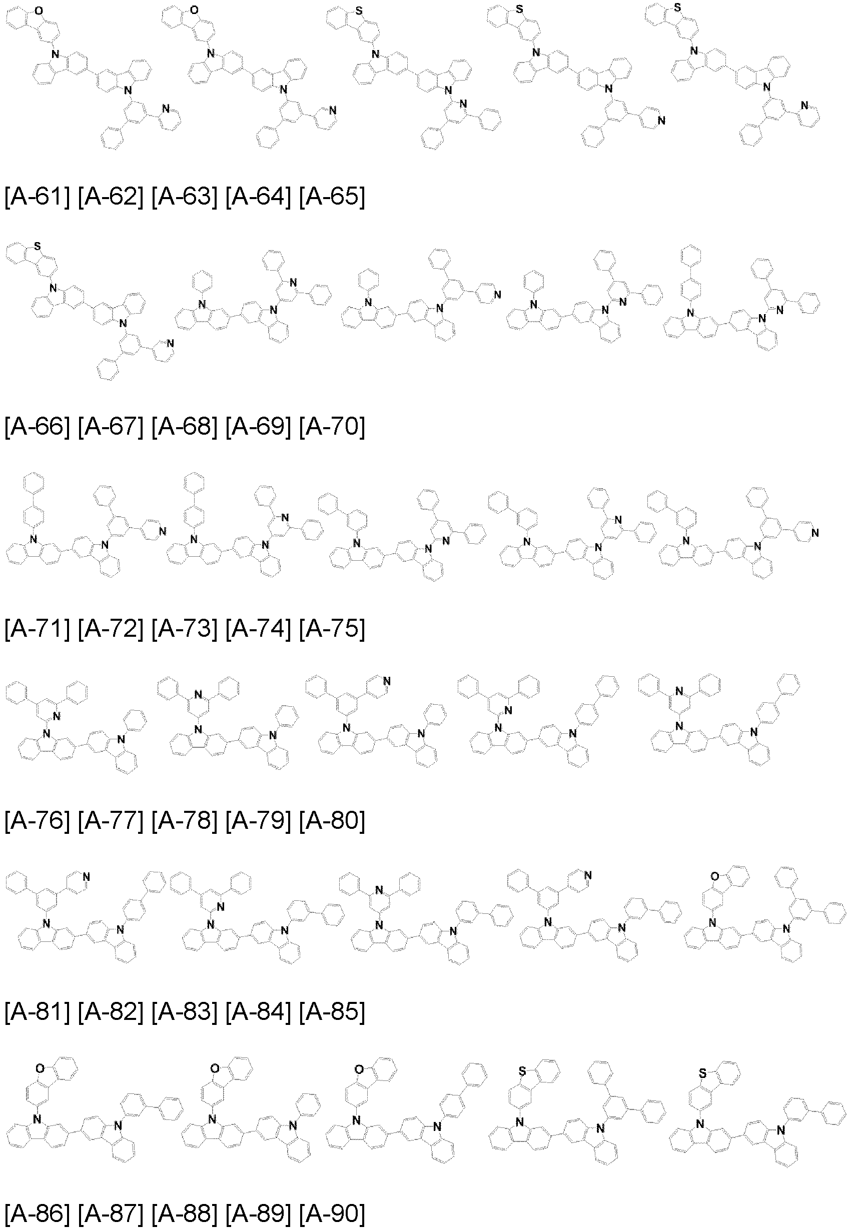

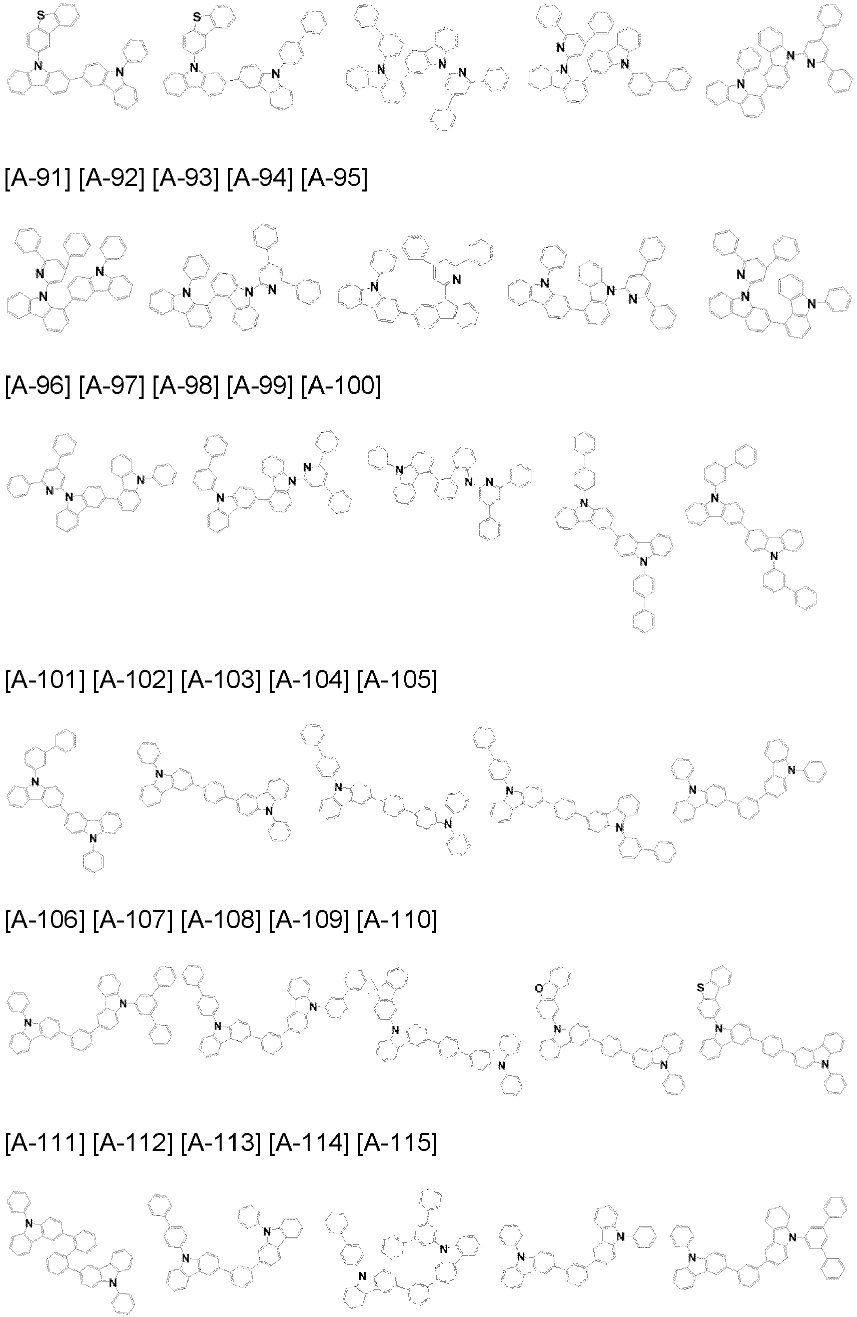

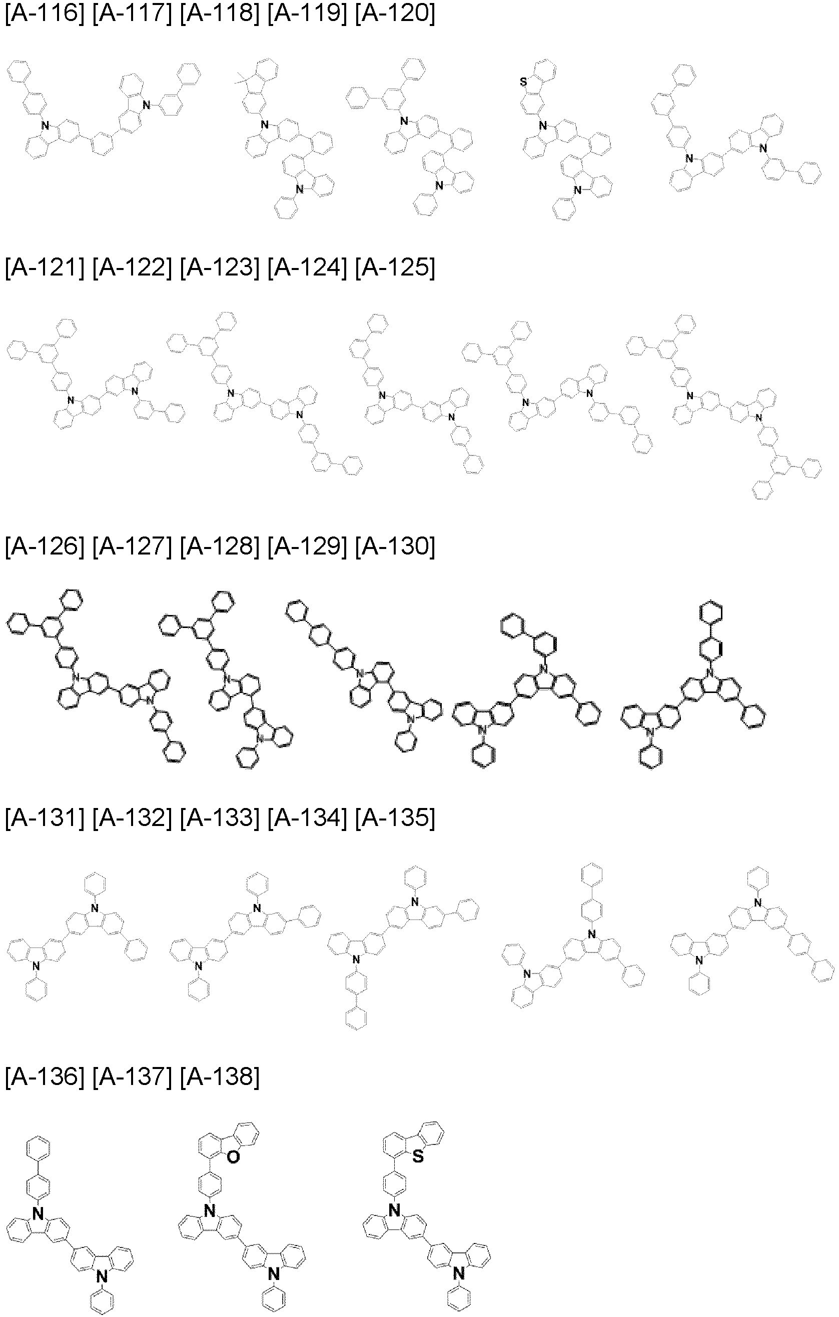

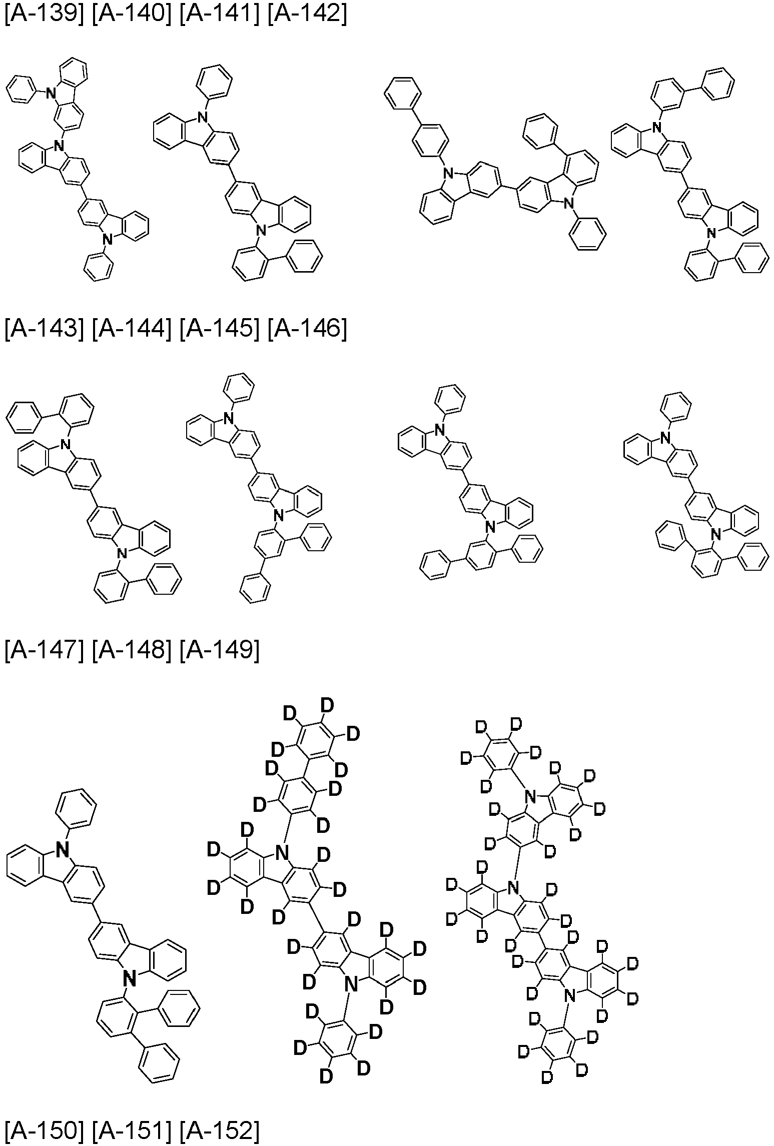

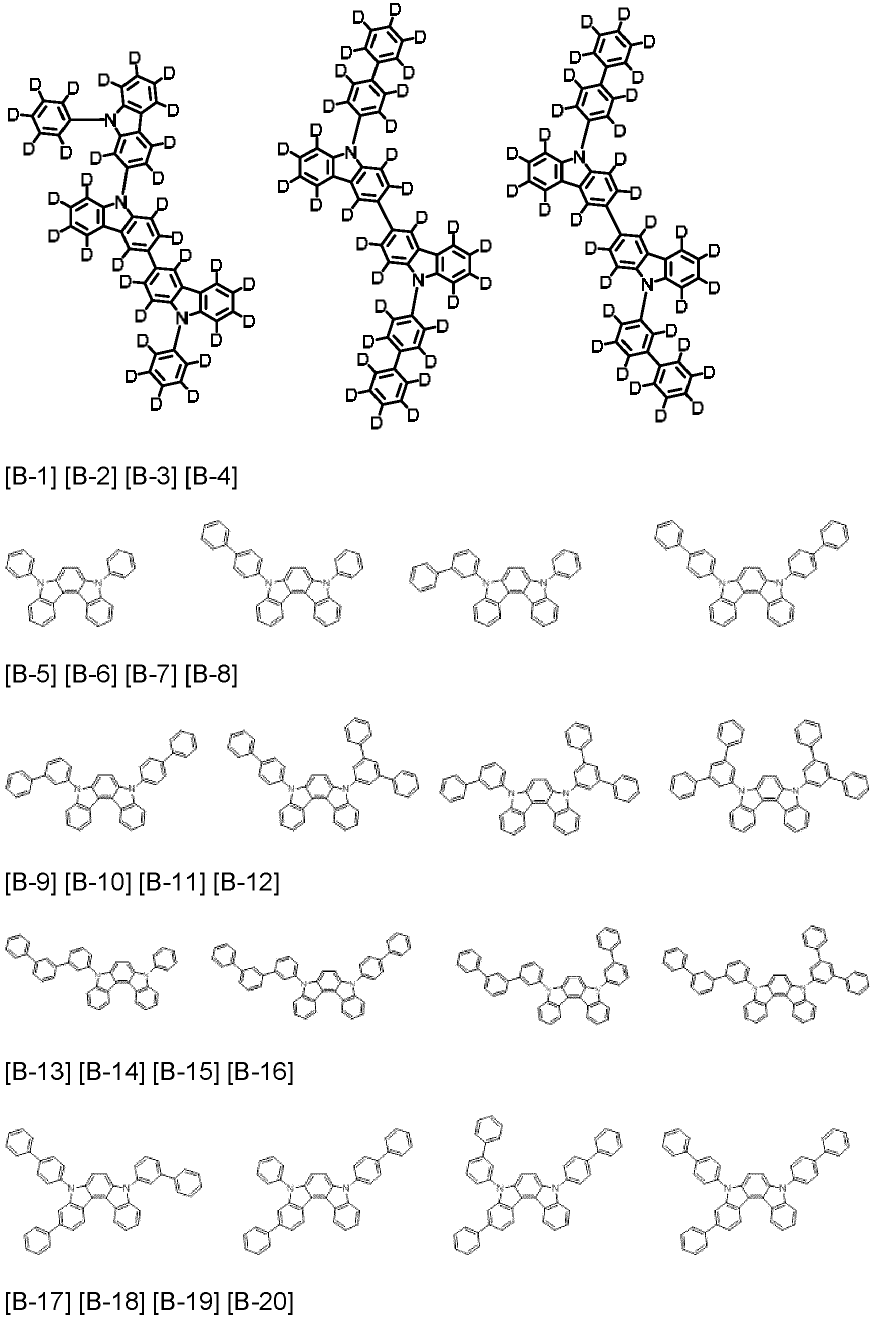

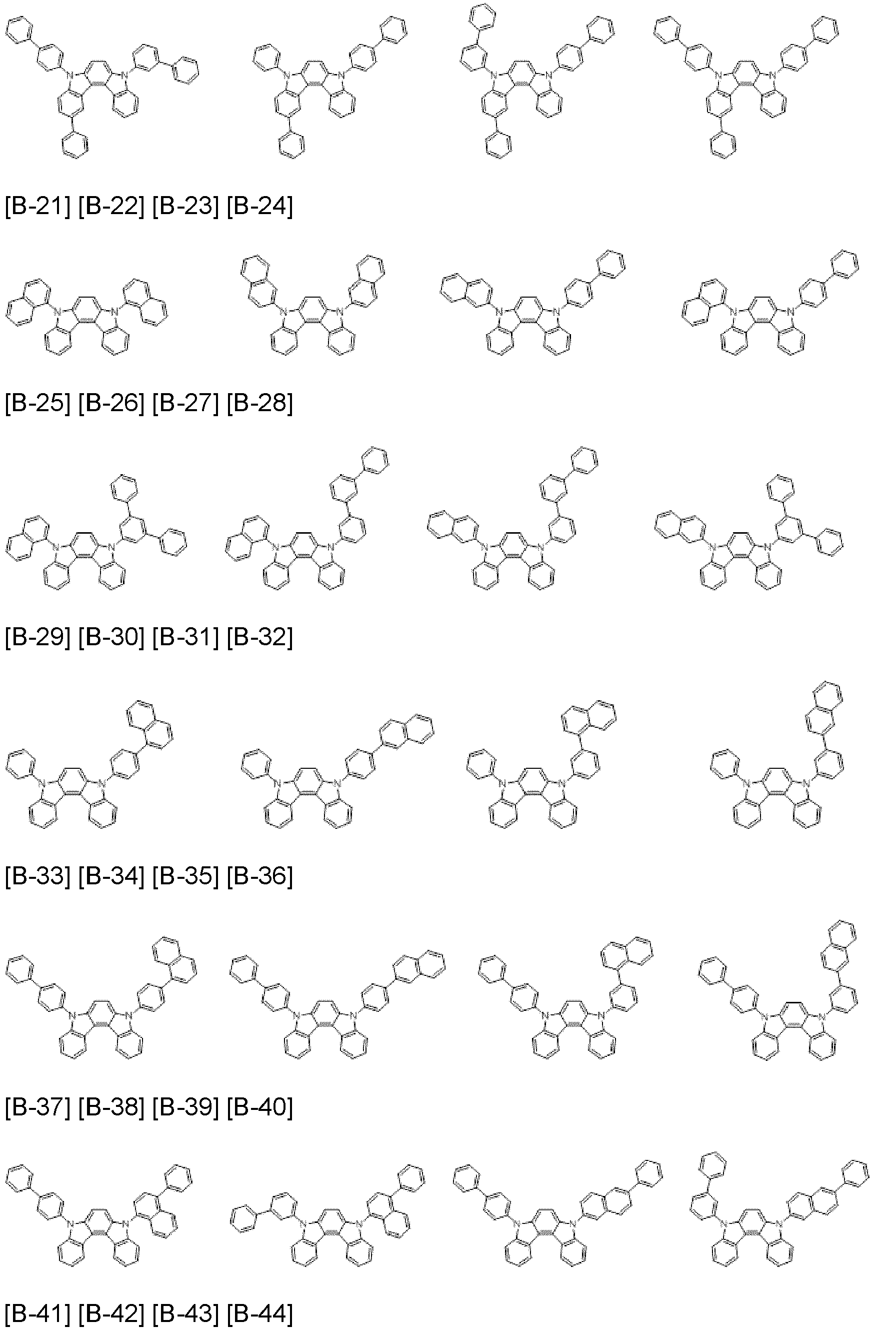

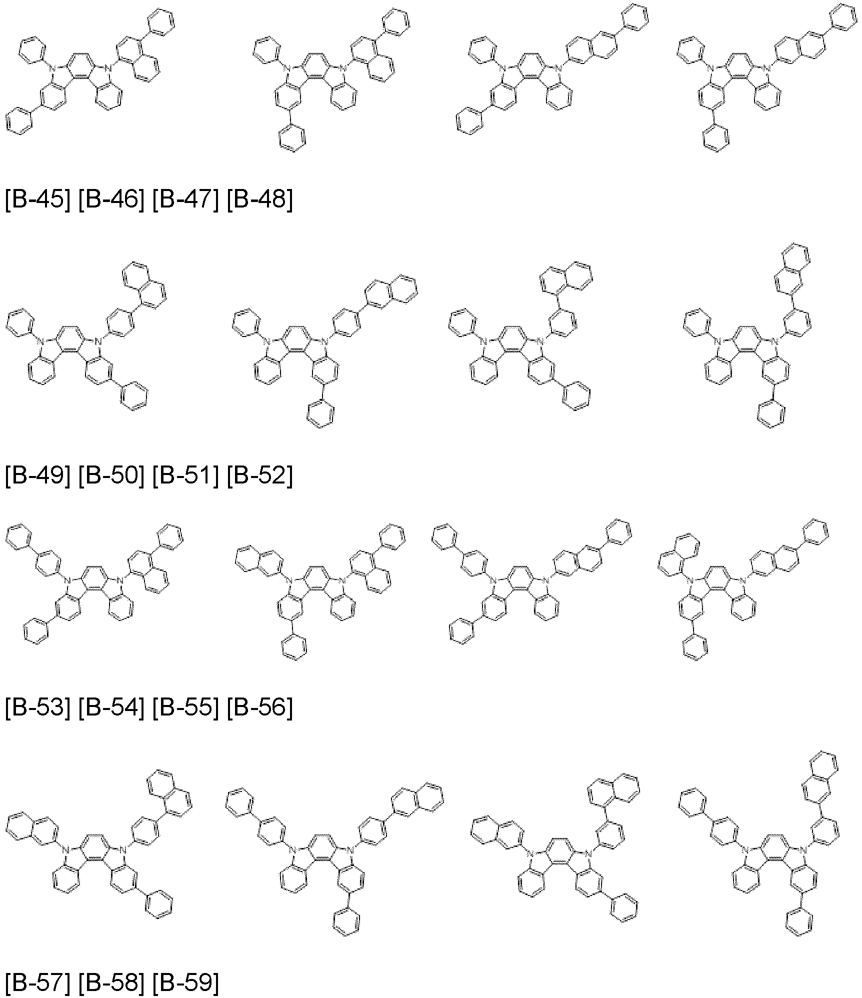

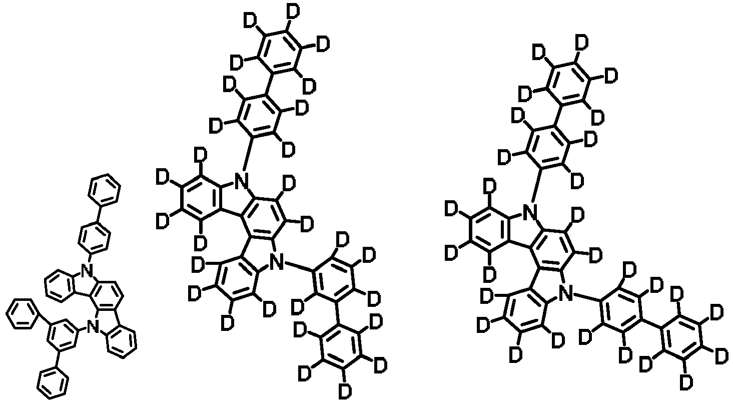

The first compound and the second compound may be for example included in a weight ratio of 1:99 to 99:1. Within the range, a desirable weight ratio may be adjusted using an electron transport capability of the first compound and a hole transport capability of the second compound to realize bipolar characteristics and thus to improve efficiency and life-span. Within the range, they may be for example included in a weight ratio of about 10:90 to 90:10, about 20:80 to 80:20, for example about 20:80 to about 70: 30, about 20:80 to about 60:40, or about 30:70 to about 60:40. As a specific example, they may be included in a weight ratio of 40:60, 50:50, or 60:40.

One or more compounds may be included in addition to the aforementioned first compound and second compound.

The aforementioned compound for an organic optoelectronic device or composition for an organic optoelectronic device may further include a dopant.

The dopant may be, for example, a phosphorescent dopant, for example, a red, green, or blue phosphorescent dopant, for example, a red or green phosphorescent dopant.

The dopant is a material mixed with the compound or composition for an organic optoelectronic device in a small amount to cause light emission, and may be generally a material such as a metal complex that emits light by multiple excitation into a triplet or more. The dopant may be, for example, an inorganic, organic, or organic-inorganic compound, and one or more types thereof may be used.

Examples of the dopant may be a phosphorescent dopant and examples of the phosphorescent dopant may be an organic metal compound including Ir, Pt, Os, Ti, Zr, Hf, Eu, Tb, Tm, Fe, Co, Ni, Ru, Rh, Pd, or a combination thereof. The phosphorescent dopant may be, for example, a compound represented by Chemical Formula Z, but is not limited thereto.

[Chemical Formula Z] L⁷MX

In Chemical Formula Z, M is a metal, and L⁷ and X are the same or different, and are a ligand to form a complex compound with M.

The M may be for example Ir, Pt, Os, Ti, Zr, Hf, Eu, Tb, Tm, Fe, Co, Ni, Ru, Rh, Pd, or a combination thereof and L¹ and X³ may be, for example a bidendate ligand.

The aforementioned compound for an organic optoelectronic device or composition for an organic optoelectronic device may be formed into a film by a dry film formation method such as chemical vapor deposition (CVD).

Hereinafter, an organic optoelectronic device including the aforementioned compound for an organic optoelectronic device or composition for an organic optoelectronic device is described.

The organic optoelectronic device may be any device to convert electrical energy into photoenergy and vice versa without particular limitation, and may be, for example an organic photoelectric device, an organic light emitting diode, an organic solar cell, and an organic photoconductor drum.

Herein, an organic light emitting diode as one example of an organic optoelectronic device is described referring to drawings.

FIGS. 1 to 4 are cross-sectional views showing organic light emitting diodes according to embodiments.

Referring to FIG. 1, an organic light emitting diode 100 according to an embodiment includes an anode 120 and a cathode 110 facing each other and an organic layer 105 disposed between the anode 120 and cathode 110.

The anode 120 may be made of a conductor having a large work function to help hole injection, and may be for example a metal, a metal oxide and/or a conductive polymer. The anode 120 may be, for example a metal such as nickel, platinum, vanadium, chromium, copper, zinc, gold, and the like or an alloy thereof; a metal oxide such as zinc oxide, indium oxide, indium tin oxide (ITO), indium zinc oxide (IZO), and the like; a combination of a metal and an oxide such as ZnO and Al or SnO₂ and Sb; a conductive polymer such as poly(3-methylthiophene), poly(3,4-(ethylene-1,2-dioxy)thiophene) (PEDOT), polypyrrole, and polyaniline, but is not limited thereto.

The cathode 110 may be made of a conductor having a small work function to help electron injection, and may be for example a metal, a metal oxide, and/or a conductive polymer. The cathode 110 may be for example a metal such as magnesium, calcium, sodium, potassium, titanium, indium, yttrium, lithium, gadolinium, aluminum silver, tin, lead, cesium, barium, and the like, or an alloy thereof; a multi-layer structure material such as LiF/Al, LiO₂/Al, LiF/Ca, LiF/Al, and BaF₂/Ca, but is not limited thereto.

The organic layer 105 may include the aforementioned compound for an organic optoelectronic device or composition for an organic optoelectronic device.

The organic layer 105 may include the light emitting layer 130, and the light emitting layer 130 may include the aforementioned compound for an organic optoelectronic device or composition for an organic optoelectronic device.

The composition for an organic optoelectronic device further including a dopant may be, for example, a green light emitting composition.

The light emitting layer 130 may include, for example, the aforementioned compound for an organic optoelectronic device or composition for an organic optoelectronic device, respectively, as a phosphorescent host.

The organic layer may further include a charge transport region in addition to the light emitting layer.

The charge transport region may be, for example, the hole transport region 140.

Referring to FIG. 2, the organic light emitting diode 200 further includes a hole transport region 140 in addition to the light emitting layer 130. The hole transport region 140 may further increase hole injection and/or hole mobility and block electrons between the anode 120 and the light emitting layer 130. Specifically, the hole transport region 140 may include a hole transport layer between the anode 120 and the light emitting layer 130, and a hole transport auxiliary layer between the light emitting layer 130 and the hole transport layer, and at least one of the compounds of Group A may be included in at least one of the hole transport layer and the hole transport auxiliary layer.

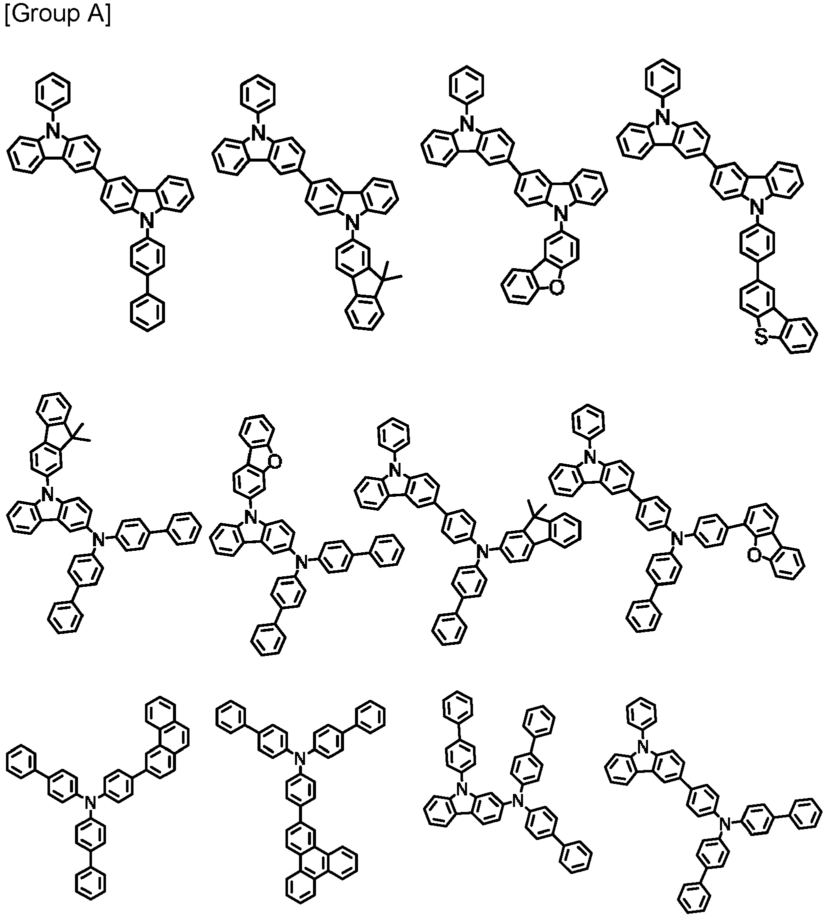

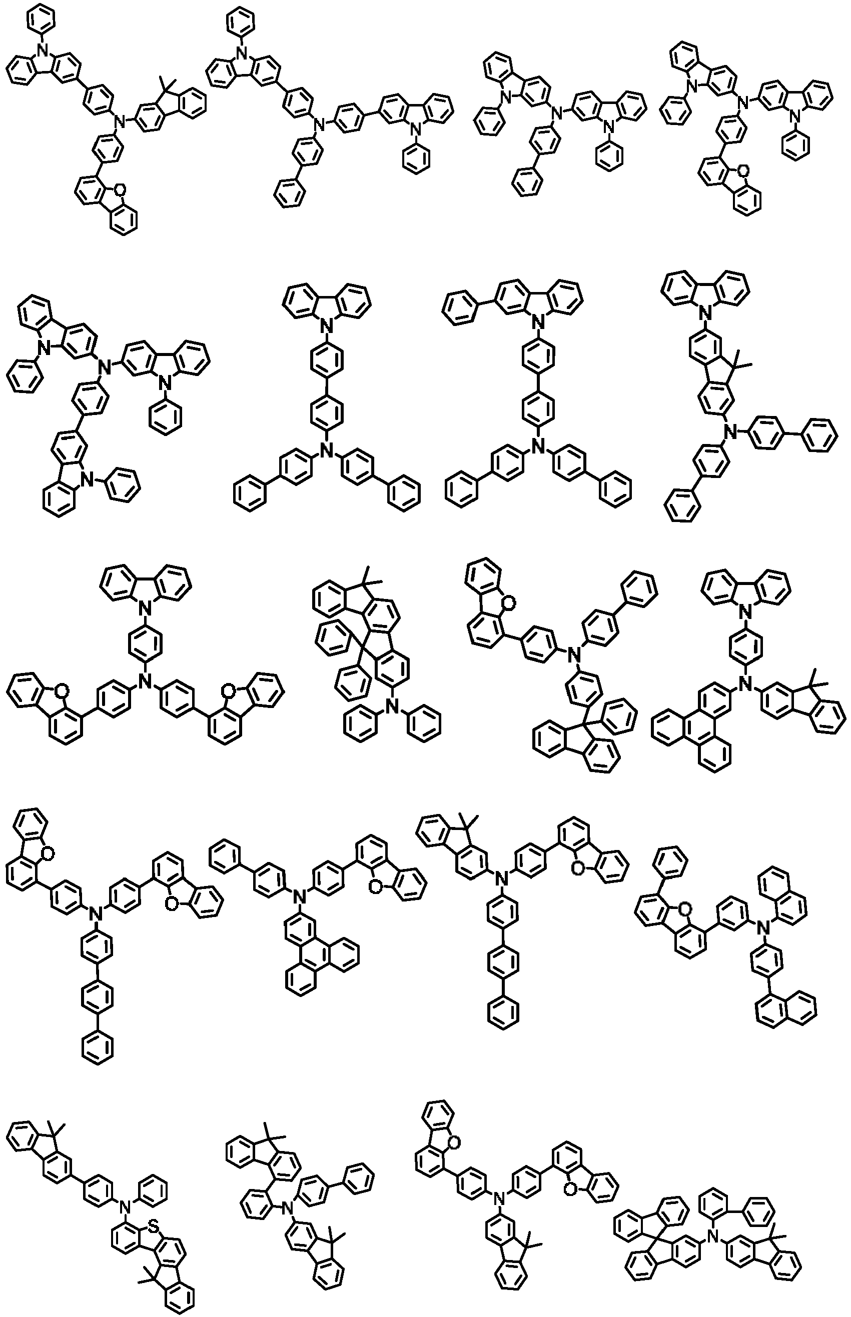

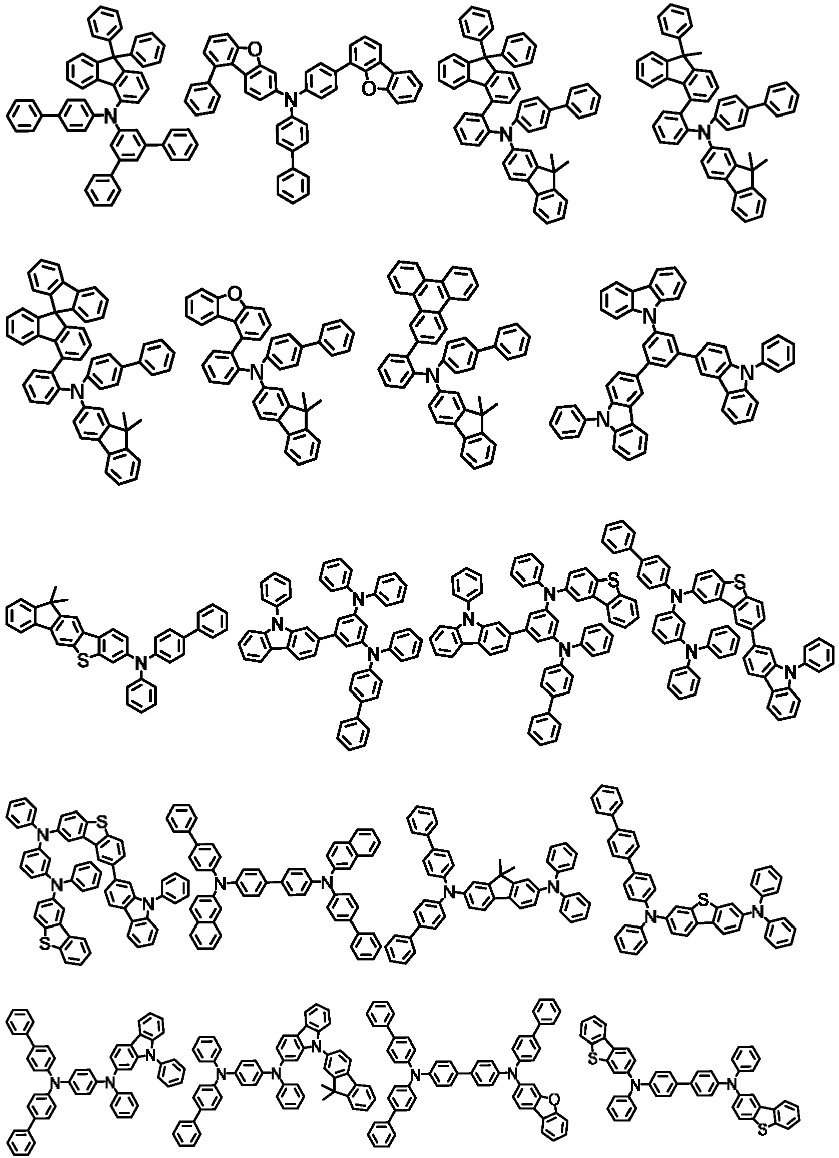

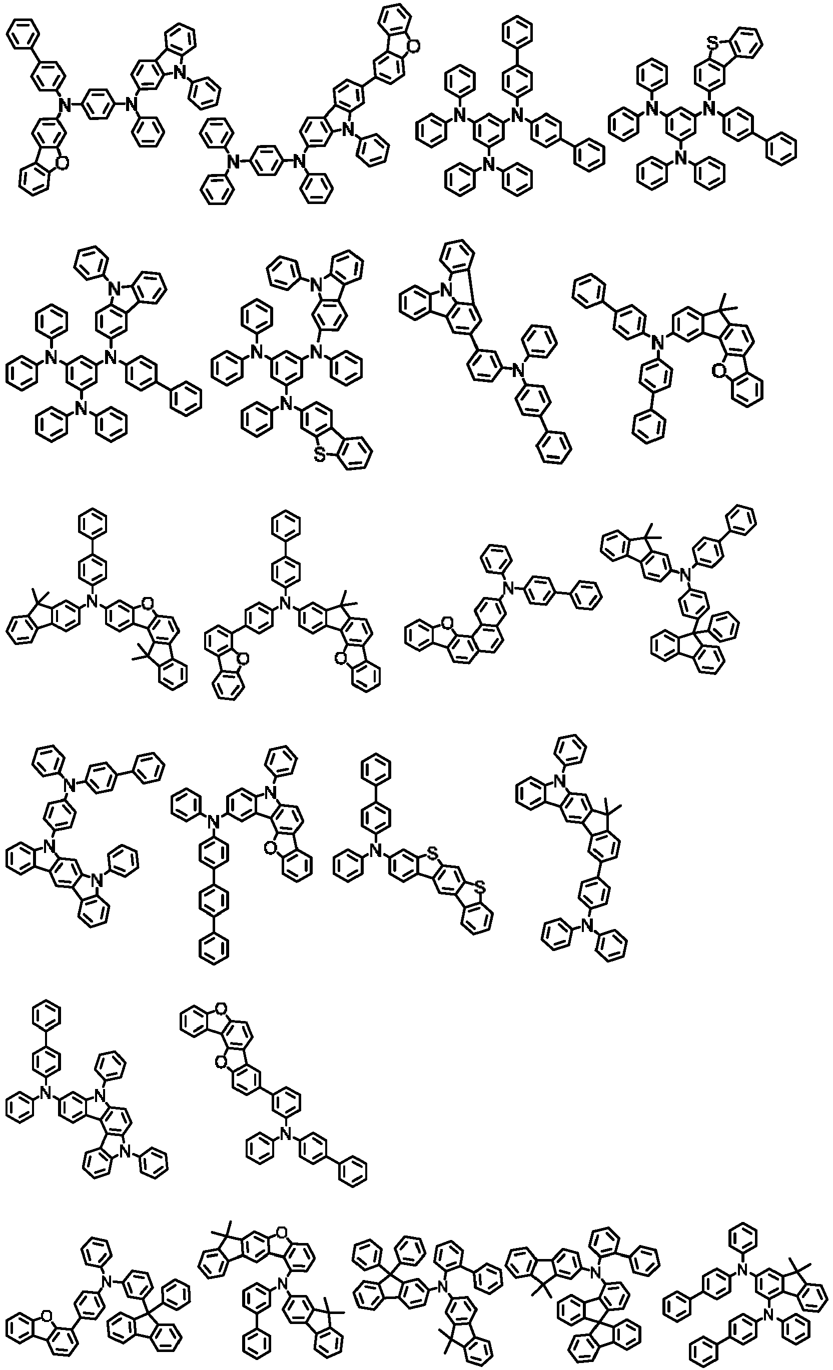

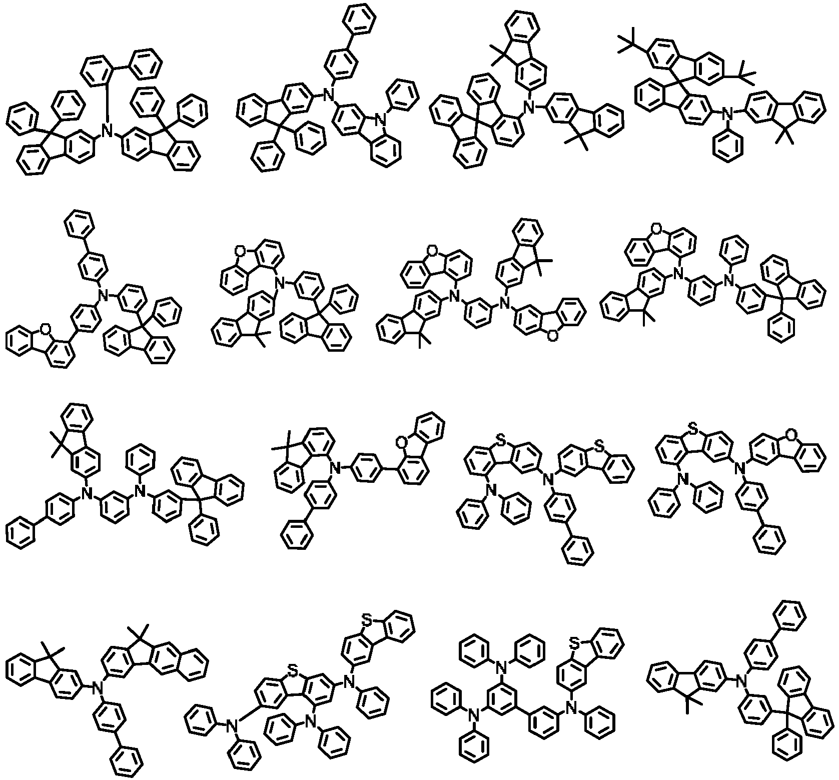

In the hole transport region, known compounds disclosed in US5061569A, JP1993-009471A, WO1995-009147A1, JP1995-126615A, JP1998-095973A, and the like and compounds similar thereto may be used in addition to the aforementioned compound.

Also, the charge transport region may be, for example, an electron transport region 150.

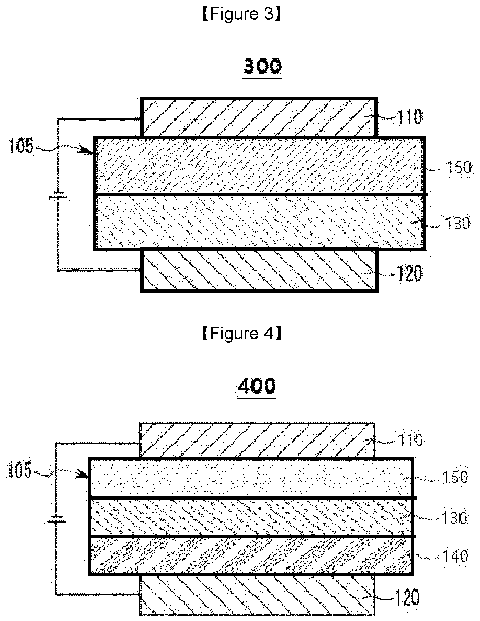

Referring to FIG. 3, the organic light emitting diode 300 further includes an electron transport region 150 in addition to the light emitting layer 130. The electron transport region 150 may further increase electron injection and/or electron mobility and block holes between the cathode 110 and the light emitting layer 130.

Specifically, the electron transport region 150 may include an electron transport layer between the cathode 110 and the light emitting layer 130, and an electron transport auxiliary layer between the light emitting layer 130 and the electron transport layer, and at least one of the compounds of Group B may be included in at least one of the electron transport layer and the electron transport auxiliary layer.

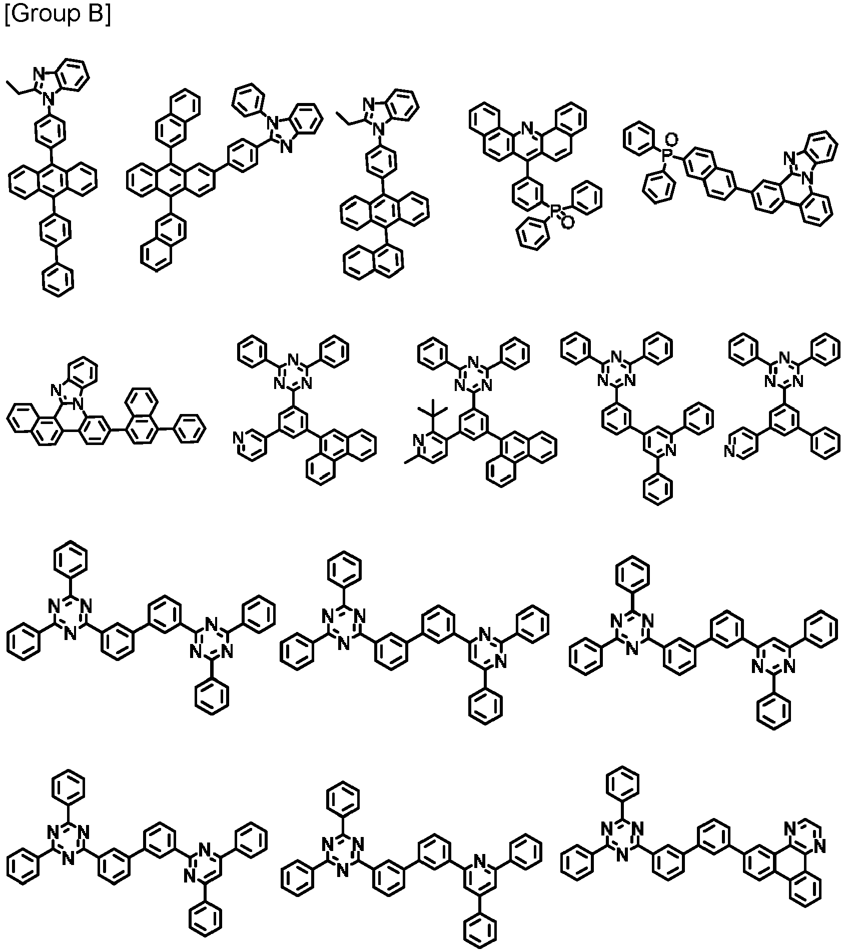

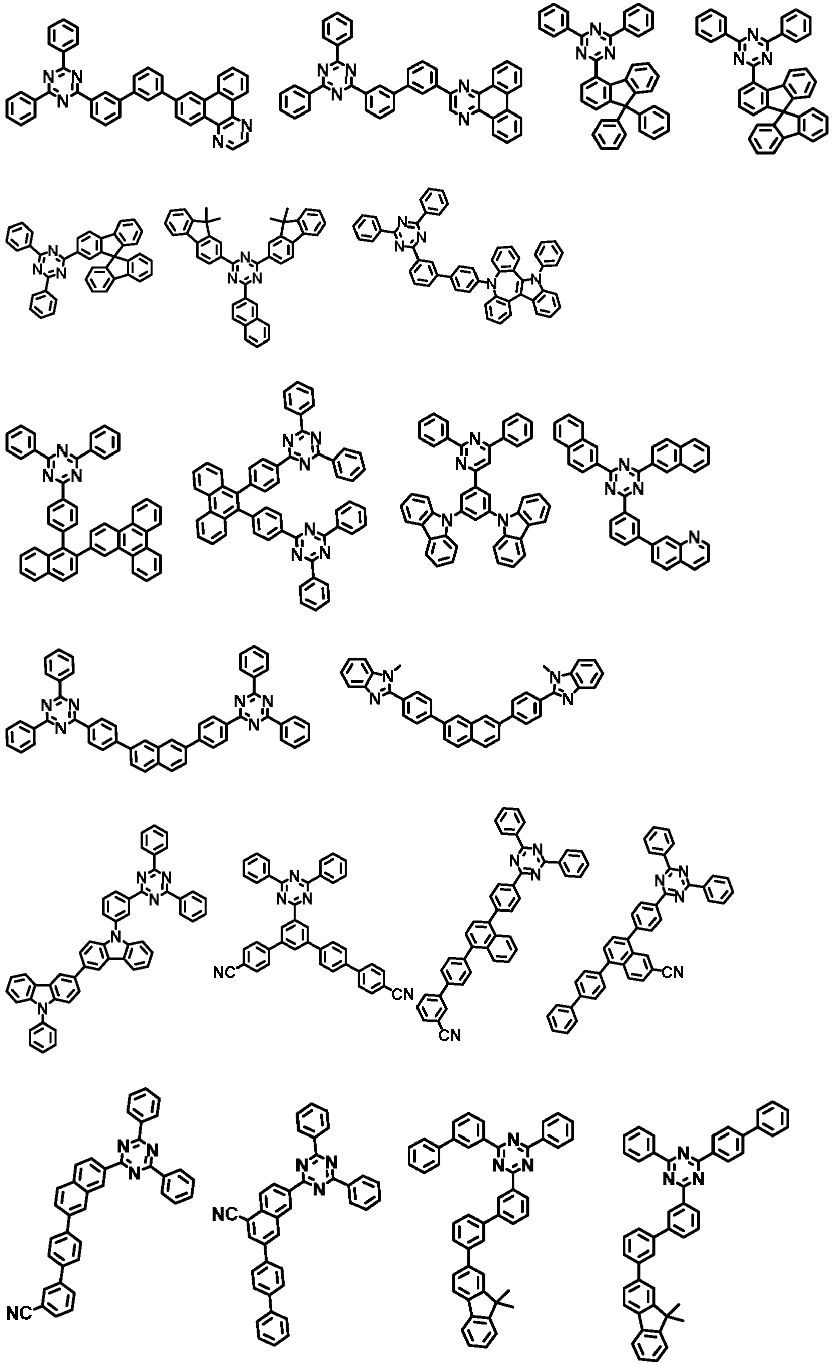

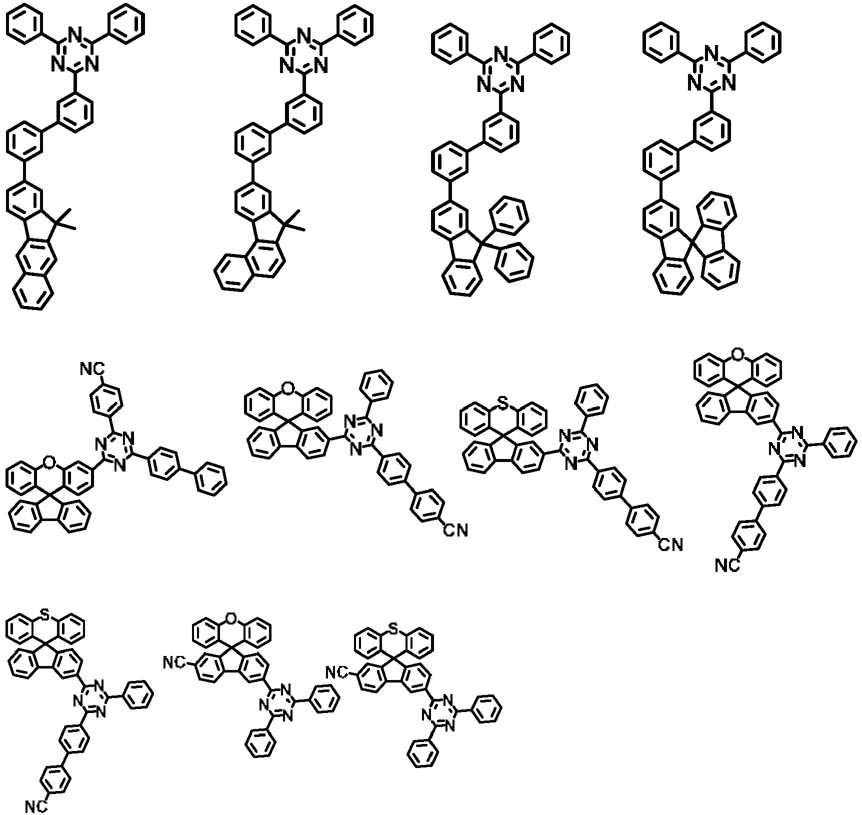

An example embodiment of the present invention may be an organic light emitting diode including the light emitting layer 130 as the organic layer 105 as shown in FIG. 1.

Another embodiment of the present invention may be an organic light emitting diode including a hole transport region 140 in addition to the light emitting layer 130 as the organic layer 105 as shown in FIG. 2.

Another embodiment of the present invention may be an organic light emitting diode including an electron transport region 150 in addition to the light emitting layer130 as the organic layer 105 as shown in FIG. 3.

Another embodiment of the present invention may be an organic light emitting diode including a hole transport region 140 and an electron transport region 150 in addition to the light emitting layer130 as the organic layer 105 as shown in FIG. 4.

Another embodiment of the present invention may be an organic light emitting diode further including an electron injection layer (not shown), a hole injection layer (not shown), etc. in addition to the light emitting layer 130 as the organic layer 105 in each of FIGS. 1 to 4.

The organic light emitting diodes 100, 200, 300, and 400 may be produced by forming an anode or a cathode on a substrate, forming an organic layer using a dry film formation method such as a vacuum deposition method (evaporation), sputtering, plasma plating, and ion plating, and forming a cathode or an anode thereon.

The organic light emitting diode may be applied to an organic light emitting display device.

### [Mode for Invention]

Hereinafter, the embodiments are illustrated in more detail with reference to examples. However, these examples are exemplary, and the present scope is not limited thereto.

Hereinafter, starting materials and reactants used in Examples and Synthesis Examples were purchased from Sigma-Aldrich Co. Ltd., TCI Inc., or Tokyo chemical industry as far as there is no particular comment or were synthesized by known methods.

### (Preparation of Compound for Organic Optoelectronic Device)

### Synthesis Example 1: Synthesis of Compound 1-3

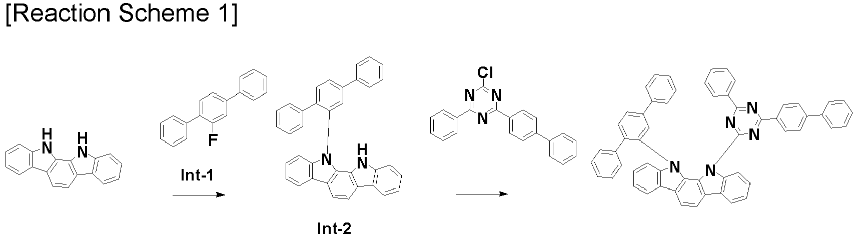

### 1st step: Synthesis of Intermediate Int-1

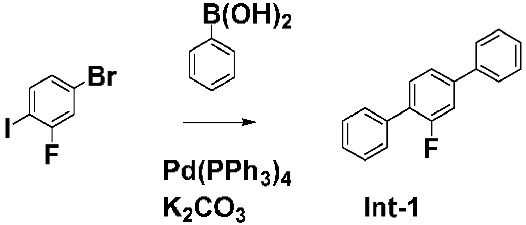

50 g (166 mmol) of 4-bromo-2-fluoro-1-iodobenzene, 43 g (349 mmol) of phenylboronic acid, 13.4 g (11.6 mmol) of Pd(PPh₃)₄, 57 g (415 mmol) of K₂CO₃, 600 ml of toluene, 200 ml of ethanol, and 200 ml of purified water were put in a reactor and then, stirred under reflux, completing a reaction. The reactant was extracted with toluene and purified water and then, crystallized with methanol, obtaining 36 g of Int-1 (2'-fluoro-1,1'-4',1"-terphenyl, a white solid).

### 2nd step: Synthesis of Intermediate Int-2

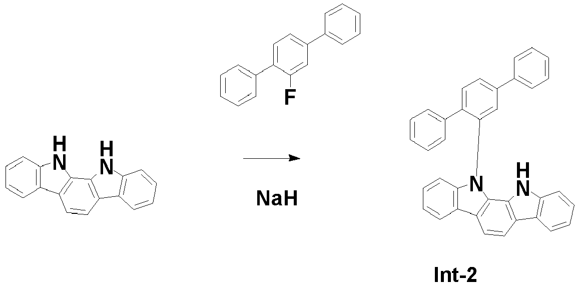

36 g (141 mmol) of 11,12-dihydroindolo[2,3-a]carbazole, 35 g (141 mmol) of Int-1, and 350 ml of N-Methyl-2-pyrrolidone were put in a reactor, and 6.8 g (170 mmol) of sodium hydride was added thereto and then, stirred under a nitrogen atmosphere. The reactant was stirred under reflux, completing a reaction. The reactant was cooled down, and purified water was added thereto to produce a solid and then, filtered and dried. The dried product was column-purified, obtaining 40 g of Intermediate Int-2 (light brown solid).

### 3rd step: Synthesis of Compound 1-3

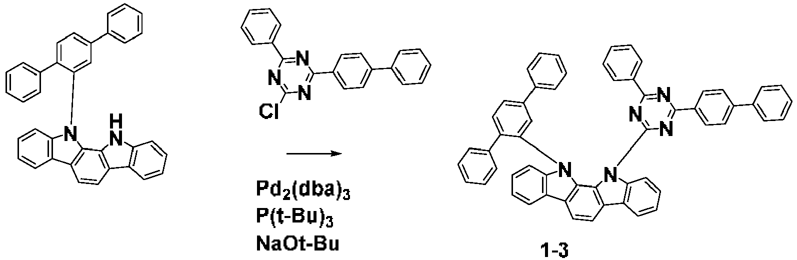

40 g (82.5 mmol) of Int-2, 34 g (100 mmol) of 2-([1,1'-biphenyl]-4-yl)-4-chloro-6-phenyl-1,3,5-triazine, 3.8 g (4.1 mmol) of Pd₂(dba)₃, 6 ml (12.3 mmol) of P(t-Bu)₃, 16 g (165 mmol) of NaOt-Bu, and 400 ml of xylene were put in a reactor and stirred under reflux, completing a reaction. The reactant was cooled down and extracted with toluene and purified water, and an organic layer therefrom was concentrated. The concentrated product was column-purified, obtaining 34 g of Compound 1-3 (light yellow solid). (LC/MS: theoretical value 791.94 g/mol, measured value: 792.49 g/mol)

### Synthesis Example 2: Synthesis of Compound A-136

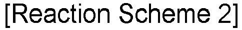

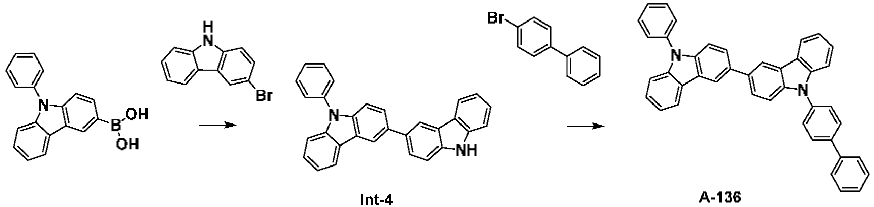

### 1st step: Synthesis of Intermediate Int-4

13.0 g (45.3 mmol) of 9-phenyl-9H-carbazole-3-boronic acid, 11.1 g (45.3 mmol) of 3-bromo-9H-carbazole, and 12.5 g (90.6 mmol) of K₂CO₃, and 2.62 g (2.2 mmol) of Pd(PPh₃)₄ were suspended in 3000 ml of THF and 110 ml of distilled water and then, stirred under reflux for 20 hours. Subsequently, the resultant was extracted with ethylacetate and distilled water, and an organic layer therefrom was concentrated under a reduced pressure. The produced solid was recrystallized with dichloromethane and hexane, obtaining 15.6 g of Intermediate Int-4.

### 2nd step: Synthesis of Compound A-136

15.6 g (38.2 mmol) of Intermediate Int-4, 8.9 g (38.2 mmol) of 4-bromobiphenyl, 1.75 g (1.9 mmol) of Pd₂(dba)₃, 2.3 g (5.7 mmol) of 50% P(t-Bu)s, and 7.3 g (76.4 mmol) of NaOt-Bu were suspended in 300 ml of toluene and stirred under reflux for 12 hours. When a reaction was completed, distilled water was added thereto and then, stirred for 30 minutes, extracted, and purified through column chromatography (hexane : dichloromethane), obtaining 15.4 g of Compound A-136. LC-Mass (theoretical value: 560.7 g/mol, measured value: M+ = 561.40 g/mol)

### Synthesis Example 3: Synthesis of Compound A-141

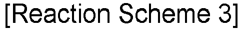

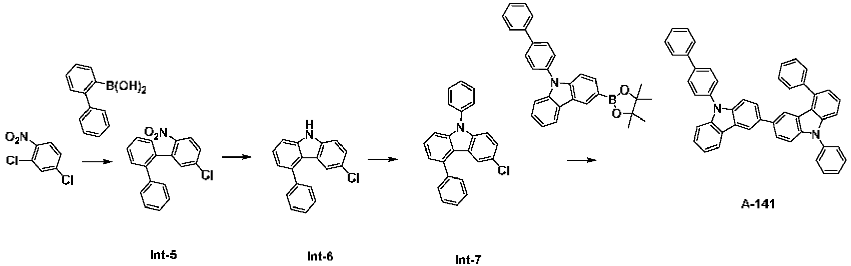

### 1st step: Synthesis of Intermediate Int-5

8.3 g (41.9 mmol) of 2-biphenylboronic acid, 8.1 g (41.9 mmol) of 2,4-dichloro-nitrobenzene, 1.9 g (1.68 mmol) of Pd(PPh₃)₄, and 14.5 g (104.7 mmol) of K₂CO₃ were suspended in 100 ml of THF and 52 ml of distilled water and then, stirred under reflux for 18 hours. Subsequently, the resultant was extracted with ethylacetate and distilled water, and an organic layer therefrom was concentrated. Then, crystals were produced by using methanol and then, filtered and dried, obtaining 8.3 g of Intermediate Int-5.

### 2nd step: Synthesis of Intermediate Int-6

8.3 g (26.8 mmol) of Int-5 and 21.1 g (80.5 mmol) of triphenylphosphine were suspended in 60 ml of 1,2-dichlorobenzene and then, stirred under reflux for 18 hours. When a reaction was completed, the 1,2-dichlorobenzene was distilled off, and the concentrated product was treated through column chromatography (hexane : dichloromethane), obtaining 3.95 g of Intermediate Int-6.

### 3rd step: Synthesis of Intermediate Int-7

3.95 g (14.2 mmol) of Intermediate Int-6, 3.05 g (15.0 mmol) of lodobenzene, 0.54 g (2.84 mmol) of Cul, 6.04 g (28.4 mmol) of K₃PO₄, and 0.86 g (14.2 mmol) of ethylenediamine were suspended in 50 ml of toluene and stirred under reflux for 18 hours. When a reaction was completed, the resultant was extracted with toluene and distilled water, and an organic layer therefrom was concentrated. The concentrated product was treated through column chromatography (hexane : dichloromethane), obtaining 2.1 g of Intermediate Int-7.

### 4th step: Synthesis of Compound A-141

2.1 g (6.0 mmol) of Intermediate Int-7, 2.7 g (6.0 mmol) of 9-biphenyl-9H-carbazole-3-boronic ester, 0.3 g (0.3 mmol) of Pd₂(dba)₃, 0.2 ml (0.9 mmol) of 50% P(t-Bu)₃, and 4.2 g (13.1 mmol) of Cs₂Co₃ were suspended in 600 ml of 1,4-dioxane and then, stirred under reflux for 18 hours. When a reaction was completed, the resultant was extracted with toluene and distilled water, and an organic layer therefrom was concentrated under a reduced pressure. The concentrated product was treated through column chromatography (hexane : dichloromethane), obtaining 2.45 g of Compound A-141. LC-Mass (theoretical value: 636.78 g/mol, measured value: M+ = 637.87 g/mol)

### Synthesis Example 4: Synthesis of Compound B-5

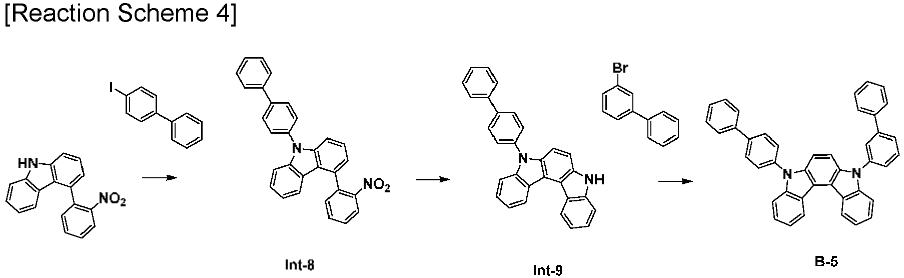

### 1st step: Synthesis of Intermediate Int-8

50 g (173 mmol) of 2-nitrophenyl-4-ylcarbazole, 53.4 g (191 mmol) of 4-iodo-biphenyl, 6.6 g (34.7 mmol) of Cul, 73.6 g (347 mmol) of K₃PO₄, and 10.5 g (173 mmol) of 1,2-ethylenediamine were suspended in 580 ml of toluene and then, stirred under reflux for 12 hours. When a reaction was completed, the resultant was extracted with toluene and distilled water, and an organic layer therefrom was concentrated under a reduced pressure. Subsequently, 1000 ml of methanol was added to the concentrated product to produce a solid and then, filtered, obtaining 68 g of Intermediate Int-8.

### 2nd step: Synthesis of Intermediate Int-9

68 g (156 mmol) of Intermediate Int-8 and 123 g (468 mmol) of triphenylphosphine were added thereto, and 520 ml of 1,2-dichlorobenzene was added thereto and then, stirred under reflux for 12 hours. When a reaction was completed, 400 ml of 1,2-dichlorobenzene was distilled off under a reduced pressure, and crystals were produced therefrom with dichloromethane and methanol and then, filtered, obtaining 49.7 g of Intermediate Int-9.

### 3rd step: Synthesis of Compound B-5

49.7 g (122 mmol) of Intermediate Int-9, 31.2 g (134 mmol) of 3-bromobiphenyl, 5.6 g (6.1 mmol) of Pd₂(dba)₃, 7.4 g (18.63 mmol) of 50% P(t-Bu)₃, and 23.4 g (243 mmol) of NaOt-Bu were suspended in 600 ml of xylene and then, stirred under reflux for 12 hours. When a reaction was completed, the resultant was extracted with toluene and distilled water, and an organic layer therefrom was concentrated under a reduced pressure. Subsequently, 500 ml of acetone was added thereto and then, stirred to produce a solid, and toluene was used for recrystallization, obtaining 47 g of Compound B-5. (LC/MS: theoretical value: 560.23 g/mol, measured value: 561.57 g/mol)

### Comparative Synthesis Example 1: Synthesis of Compound Host1

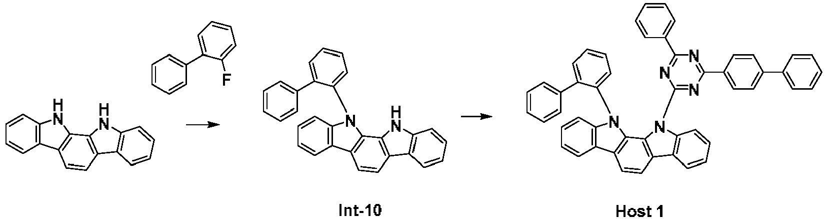

### 1st step: Synthesis of Compound Int-10

The same method as in the 2nd step of Synthesis Example 1 was used for synthesis.

6 g (35.1 mmol) of 2-fluoro-biphenyl, 9 g (35.1 mmol) of 11,12-dihydroindolo[2,3-a]carbazole, 35 g (0.141 mol) of Int-1, and 35 ml of N-methyl-2-pyrrolidone were put in a reactor, and 1.7 g (42 mmol) of sodium hydride was added thereto and then, stirred under a nitrogen atmosphere. The reactant was stirred under reflux, completing a reaction. The reactant was cooled down, and purified water was added thereto to produce a solid and then, filtered and dried. The dried product was column-purified, obtaining 7.3 g of Int-10 (light brown solid).

### 2nd step: Synthesis of Compound Host 1

8.9 g of Host 1 was synthesized and purified in the same method as the 3rd step of Synthesis Example 1 except that 7.3 g of Int-10 was used instead of Int-2. (LC/MS: theoretical value: 715.84 g/mol, measured value: 716.30 g/mol)

### Comparative Synthesis Example 2: Synthesis of Compound Host2

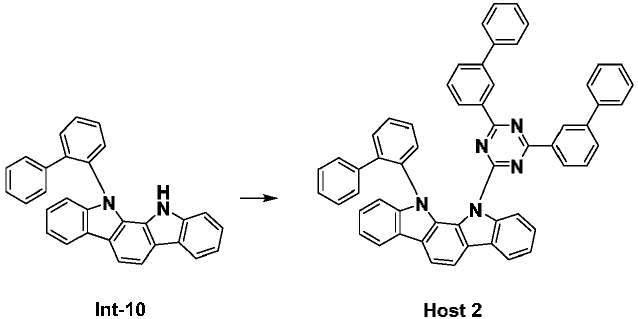

4 g of Host 2 was synthesized and purified in the same method as the 3rd step of Synthesis Example 1 except that 2-chloro-4,6-di(biphenyl-3-yl)-1,3,5-triazine was used instead of the 2-([1,1'-biphenyl]-4-yl)-4-chloro-6-phenyl-1,3,5-triazine. (LC/MS: theoretical value: 791.94 g/mol, measured value: 792.20 g/mol)

### Comparative Synthesis Example 3: Synthesis of Compound Host 3

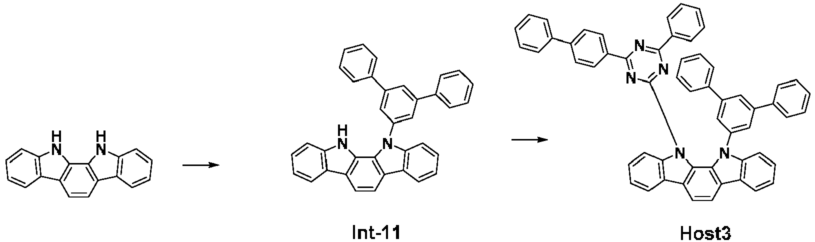

### 1st step: Synthesis of Compound Int-11

50 g (161.7 mmol) of 1-bromo-3,5-diphenylbenzene, 41.5 g (161.7 mmol) of 11,12-dihydroindolo[2,3-a]carbazole, 3.0 g (3.23 mmol) of Pd₂(dba)₃, 6.5 g (16.2 mmol) of 50% P(t-Bu)₃, and 15.54 g (161.7 mmol) of NaOt-Bu were suspended in 500 ml of xylene and then, stirred under reflux for 12 hours. When a reaction was completed, the resultant was extracted with toluene and distilled water, and an organic layer therefrom was concentrated under a reduced pressure. The concentrated product was column-purified, obtaining 54.8 g of Int-11.

### 2nd step: Synthesis of Compound Host 3

48.3 g of Host 3 was synthesized and purified in the same method as the 3rd step of Synthesis Example 1 except that 39.4 g of Int-11 and 2-([1,1'-biphenyl]-4-yl)-4-chloro-6-phenyl-1,3,5-triazine were reacted. (LC/MS: theoretical value: 791.94 g/mol, measured value: 792.24 g/mol)

### (Manufacture of Organic Light Emitting Diode)

### Example 1

The glass substrate coated with ITO (Indium tin oxide) was washed with distilled water and ultrasonic waves. After washing with the distilled water, the glass substrate was washed with a solvent such as isopropyl alcohol, acetone, methanol, and the like ultrasonically and dried and then, moved to a plasma cleaner, cleaned by using oxygen plasma for 10 minutes, and moved to a vacuum depositor. This obtained ITO transparent electrode was used as an anode, Compound A doped with 3% NDP-9 (available from Novaled) was vacuum-deposited on the ITO substrate to form a 50 Å-thick hole injection layer, and Compound A was deposited on the hole injection layer to form a 1350 Å-thick hole transport layer, and Compound B was deposited on the hole transport layer to form a 350 Å-thick hole transport auxiliary layer. On the hole transport auxiliary layer, 400 Å-thick light emitting layer was formed by using Compound 1-3 of Synthesis Example 1 as a host and doping 10 wt% of PhGD as a dopant. Subsequently, Compound C was deposited on the light emitting layer to form a 50 Å-thick electron transport auxiliary layer, and Compound D and Liq were simultaneously vacuum-deposited at a weight ratio of 1:1 to form a 300 Å-thick electron transport layer. LiQ (15 Å) and Al (1200 Å) were sequentially vacuum-deposited on the electron transport layer to form a cathode, thereby manufacturing an organic light emitting diode.

ITO/Compound A (doped with 3% NDP-9, 50 A)/Compound A (1350 A)/Compound B (350 Å)/EML [Compound 1-3:PhGD=90:10 wt%)] (400 A)/Compound C(50 A)/Compound D:LiQ (300 Å)/LiQ(15 Å)/Al(1200 Å).
Compound A: N-(biphenyl-4-yl)-9,9-dimethyl-N-(4-(9-phenyl-9H-carbazol-3-yl)phenyl)-9H-fluoren-2-amine
Compound B: N,N-bis(9,9-dimethyl-9H-fluoren-4-yl)-9,9-spirobi(fluorene)-2-amine
Compound C: 2-(3-(3-(9,9-dimethyl-9H-fluoren-2-yl)phenyl)phenyl)-4,6-diphenyl-1,3,5-triazine
Compound D: 8-(4-(4,6-di(naphthalen-2-yl)-1,3,5-triazin-2-yl)phenyl)quinolone

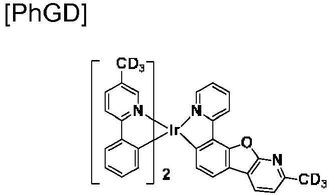

### Example 2

An organic light emitting diode was manufactured in the same manner as in Example 1 except that Compound 1 and Compound A-136 of Synthesis Example 2 were mixed in a weight ratio of 3:7 instead of Compound 1.

### Comparative Example 1

An organic light emitting diode was manufactured in the same manner as in Example 1 except that Host 1 was used instead of Compound 1.

### Comparative Example 2

An organic light emitting diode was manufactured in the same manner as in Example 1 except that Host 2 was used instead of Compound 1.

### Comparative Example 3

An organic light emitting diode was manufactured in the same manner as in Example 1 except that Host 3 was used instead of Compound 1.

### Comparative Example 4

An organic light emitting diode was manufactured in the same manner as in Example 1 except that Host 1 of Comparative Synthesis Example 1 and Compound A-136 were mixed in a weight ratio of 3:7 instead of Compound 1.

### Comparative Example 5

An organic light emitting diode was manufactured in the same manner as in Example 1 except that Host 2 of Comparative Synthesis Example 2 and Compound A-136 were mixed in a weight ratio of 3:7 instead of Compound 1.

### Comparative Example 6

An organic light emitting diode was manufactured in the same manner as in Example 1 except that Host 3 of Comparative Synthesis Example 3 and Compound A-141 were mixed in a weight ratio of 35:65 instead of Compound 1.

### Evaluation

Driving voltages, luminous efficiency, and life-span characteristics of the organic light emitting diodes according to Examples 1 and 2 and Comparative Examples 1 to 6 were evaluated.

Specific measurement methods are as follows, and the results are shown in Table 1.

### (1) Measurement of Current Density Change Depending on Voltage Change

The obtained organic light emitting diodes were measured regarding a current value flowing in the unit device, while increasing the voltage from 0 V to 10 V using a current-voltage meter (Keithley 2400), and the measured current value was divided by area to provide the results.

### (2) Measurement of Luminance Change Depending on Voltage Change

Luminance was measured by using a luminance meter (Minolta Cs-1000A), while the voltage of the organic light emitting diodes was increased from 0 V to 10 V.

### (3) Measurement of Luminous Efficiency

Current efficiency (cd/A) at the same current density (10 mA/cm²) were calculated by using the luminance and current density from the items (1) and (2), and a voltage.

### (4) Measurement of Life-span

The results were obtained by measuring a time when current efficiency (cd/A) was decreased down to 97%, while luminance (cd/m²) was maintained to be 18000 cd/m².

### (5) Measurement of Driving Voltage

The driving voltage of each diode was measured at 15mA/cm² using a current-voltmeter (Keithley 2400) to obtain the results.

The values shown in Table 1 are relative values based on the values of Example 1, respectively.

The values shown in Table 2 are relative values based on the values of Example 2, respectively.

**(Table 1)**

| Nos. | Compound | Driving voltage (V)% | Color (EL color) | Luminous efficiency (%) | Life-span (T97@18K) (%) |
|---|---|---|---|---|---|
| Example 1 | 1-3 | 100% | Green | 100% | 100% |
| Compar ative Example 1 | Host1 | 101.0% | Green | 96.4% | 88.2% |
| Compar ative Example 2 | Host2 | 107.6% | Green | 99.2% | 94.1% |
| Compar ative Example 3 | Host3 | 100.8% | Green | 82.7% | 88.2% |

Referring to Table 1, the driving voltage, luminous efficiency, and life-span characteristic of the organic light emitting diode according to Example 1 was significantly improved, compared with the organic light emitting diodes according to Comparative Examples 1 to 3.

**(Table 2)**

| No. | Compound (first host) | Compound (second host) | Color (EL color) | Luminous efficiency (cd/A) | Life-span (T97@18K) (h) |
|---|---|---|---|---|---|
| Example 2 | 1-3 | A-136 | Green | 100% | 100% |
| Compar ative Example 4 | Host1 | A-136 | Green | 98.3% | 95.3% |
| Compar ative Example 5 | Host2 | A-136 | Green | 95.7% | 93.0% |
| Compar ative Example 6 | Host3 | A-141 | Green | 99.1% | 55.8% |

Referring to Table 2, the organic light emitting diode according to Example 2 exhibited significantly improved luminous efficiency and life-span characteristics compared with the organic light emitting diodes according to Comparative Examples 4 to 6.

While this invention has been described in connection with what is presently considered to be practical embodiments, it is to be understood that the invention is not limited to the disclosed embodiments, but, on the contrary, is intended to cover various modifications and equivalent arrangements included within the spirit and scope of the appended claims.

## Claims

1. A compound for an organic optoelectronic device represented by a combination of Chemical Formula 1 and Chemical Formula 2: 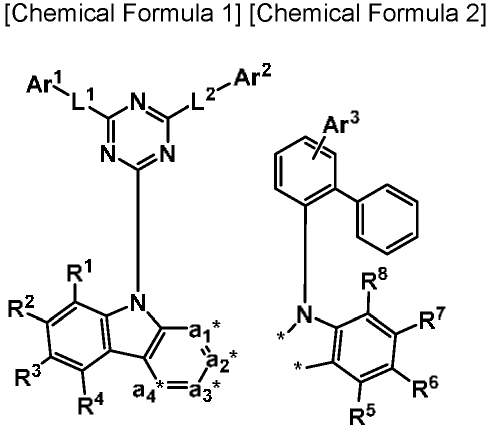 wherein, in Chemical Formula 1 and Chemical Formula 2,
adjacent two of a₁* to a₄* of Chemical Formula 1 are each independently linking carbon linked to * in Chemical Formula 2,
the rest two of a₁* to a₄* of Chemical Formula 1 not linked to * in Chemical Formula 2 are each independently C-L^{a}-R^{a},
Ar¹ and Ar² are each independently a substituted or unsubstituted C6 to C30 aryl group or a substituted or unsubstituted C2 to C30 heterocyclic group,
Ar³ is a substituted or unsubstituted C6 to C20 aryl group,
L^{a}, L¹, and L² are each independently a single bond, a substituted or unsubstituted C6 to C30 arylene group or a substituted or unsubstituted C2 to C30 heterocyclic group, and
R^{a}, and R¹ to R⁸ are each independently hydrogen, deuterium, a halogen, a substituted or unsubstituted C1 to C30 alkyl group, or a substituted or unsubstituted C6 to C30 aryl group.

2. The compound of claim 1, wherein the combination of Chemical Formula 1 and Chemical Formula 2 is represented by one of Chemical Formula 1A to Chemical Formula 1F: 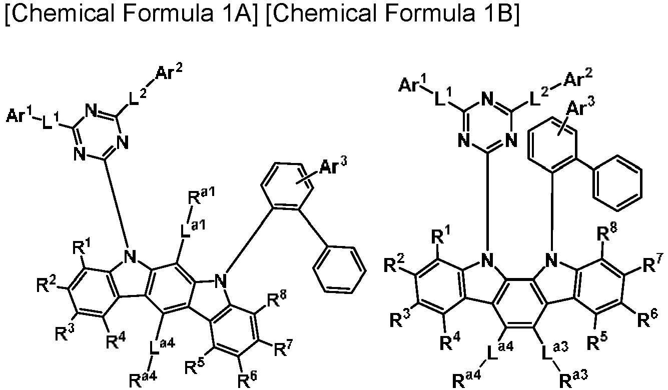 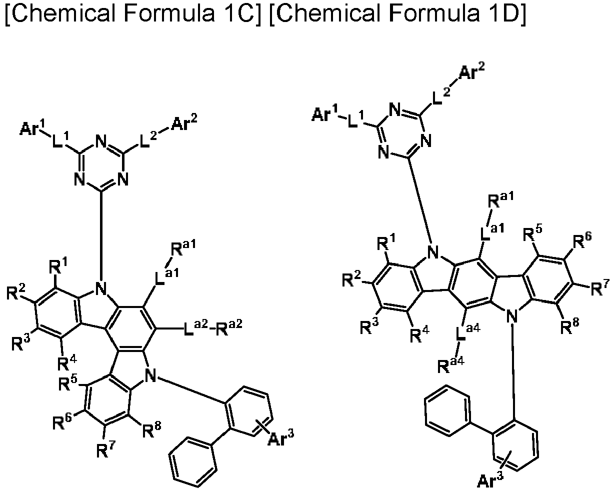 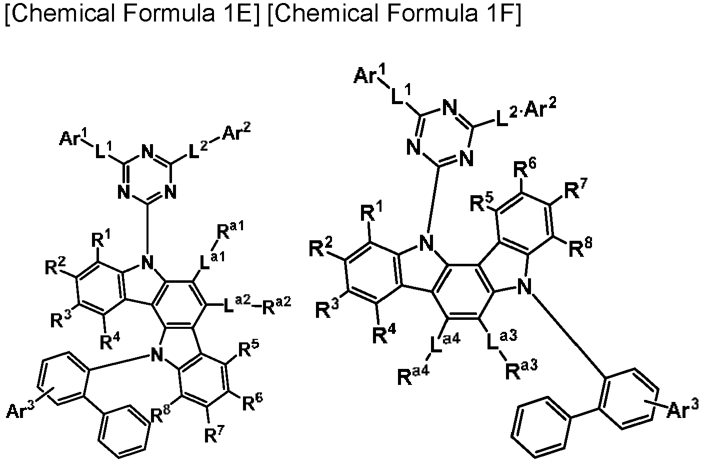 wherein, in Chemical Formula 1A to Chemical Formula 1F,
Ar¹ to Ar³, L¹, L², and R¹ to R⁸ are the same as defined in claim 1,
L^{a1} to L^{a4} are the same as L^{a} defined in claim 1, and
R^{a1} to R^{a4} are the same as R^{a} defined in claim 1.

3. The compound of claim 2, wherein
the combination of Chemical Formula 1 and Chemical Formula 2 is represented by Chemical Formula 1B, and
Chemical Formula 1B is represented by one of Chemical Formula 1B-1 to Chemical Formula 1B-4: 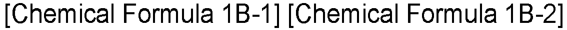 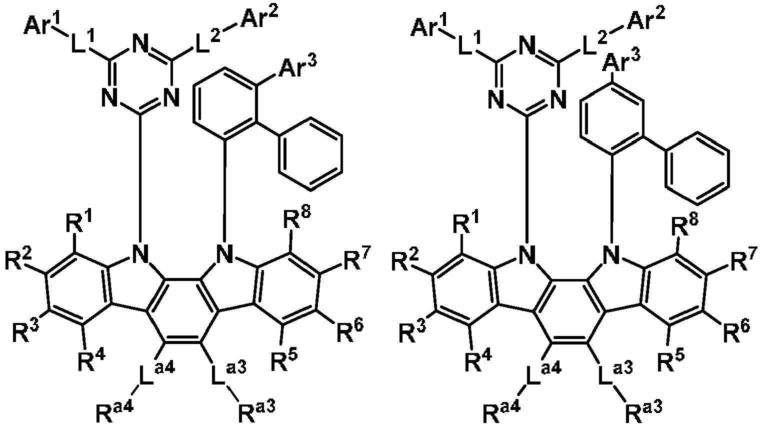 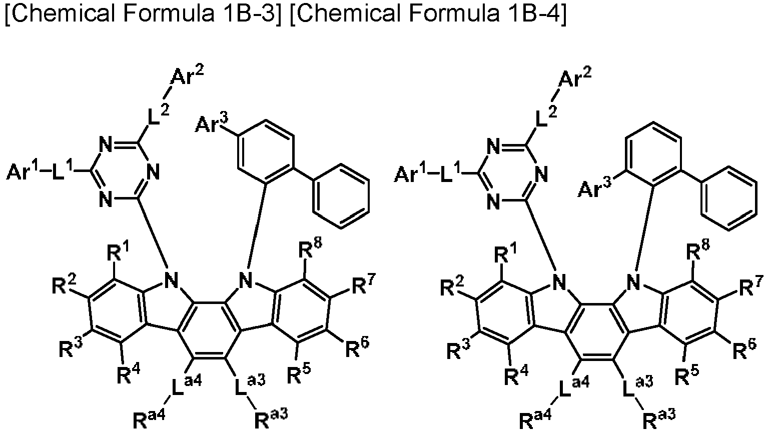
wherein, in Chemical Formula 1B-1 to Chemical Formula 1B-4,
Ar¹ to Ar³, L¹, L², L^{a3}, L^{a4}, and R¹ to R⁸ are the same as defined in claim 2.

4. The compound of claim 1, wherein Ar³ is a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, or a substituted or unsubstituted triphenylene group.

5. The compound of claim 1, wherein Ar¹ and Ar² are each independently a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted triphenylene group, a substituted or unsubstituted fluorenyl group, a substituted or unsubstituted dibenzofuranyl group, a substituted or unsubstituted dibenzothiophenyl group, or a substituted or unsubstituted dibenzosilolyl group.

6. The compound of claim 1, wherein -L¹-Ar¹ and -L²-Ar² are each independently selected from substituents of Group I : 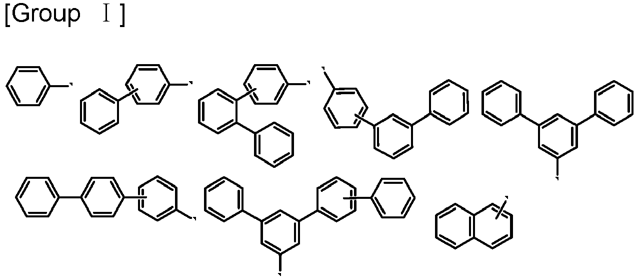 wherein, in Group I , * is a linking point.

7. The compound of claim 1, which is one of compounds of Group 1:

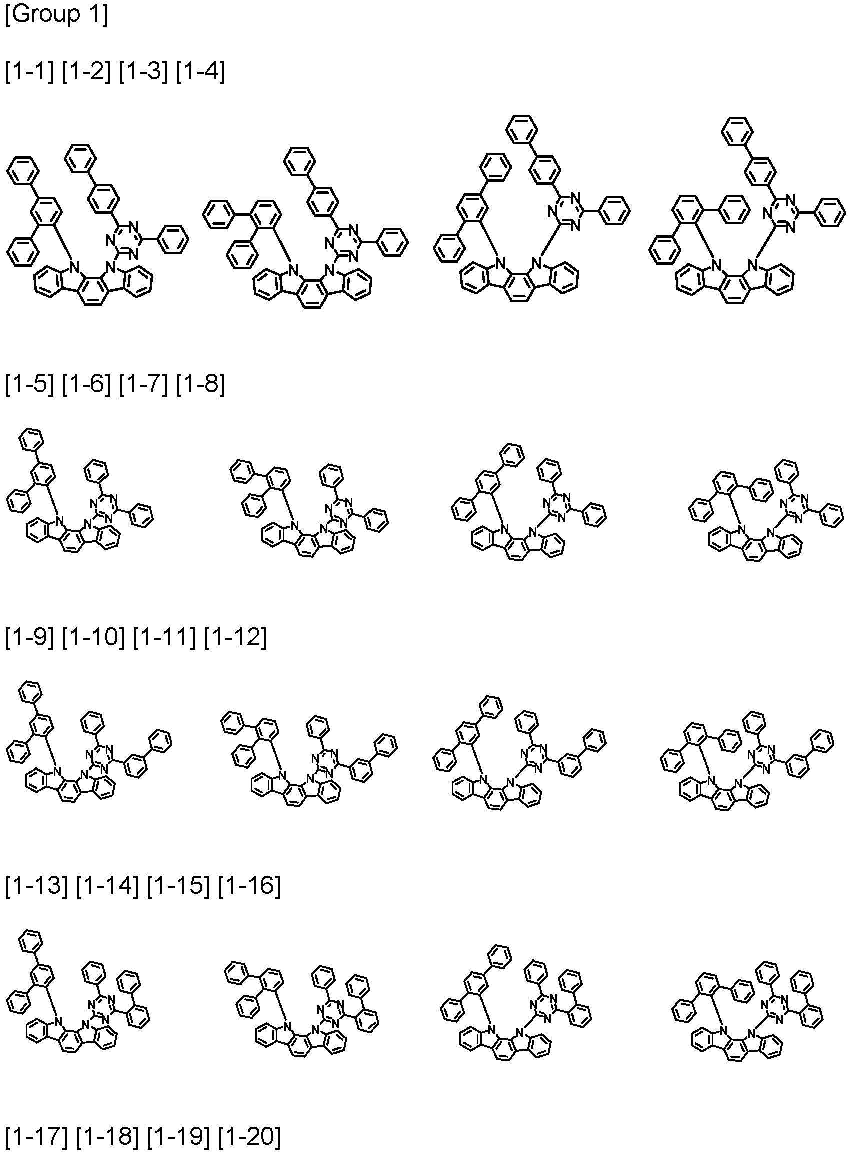

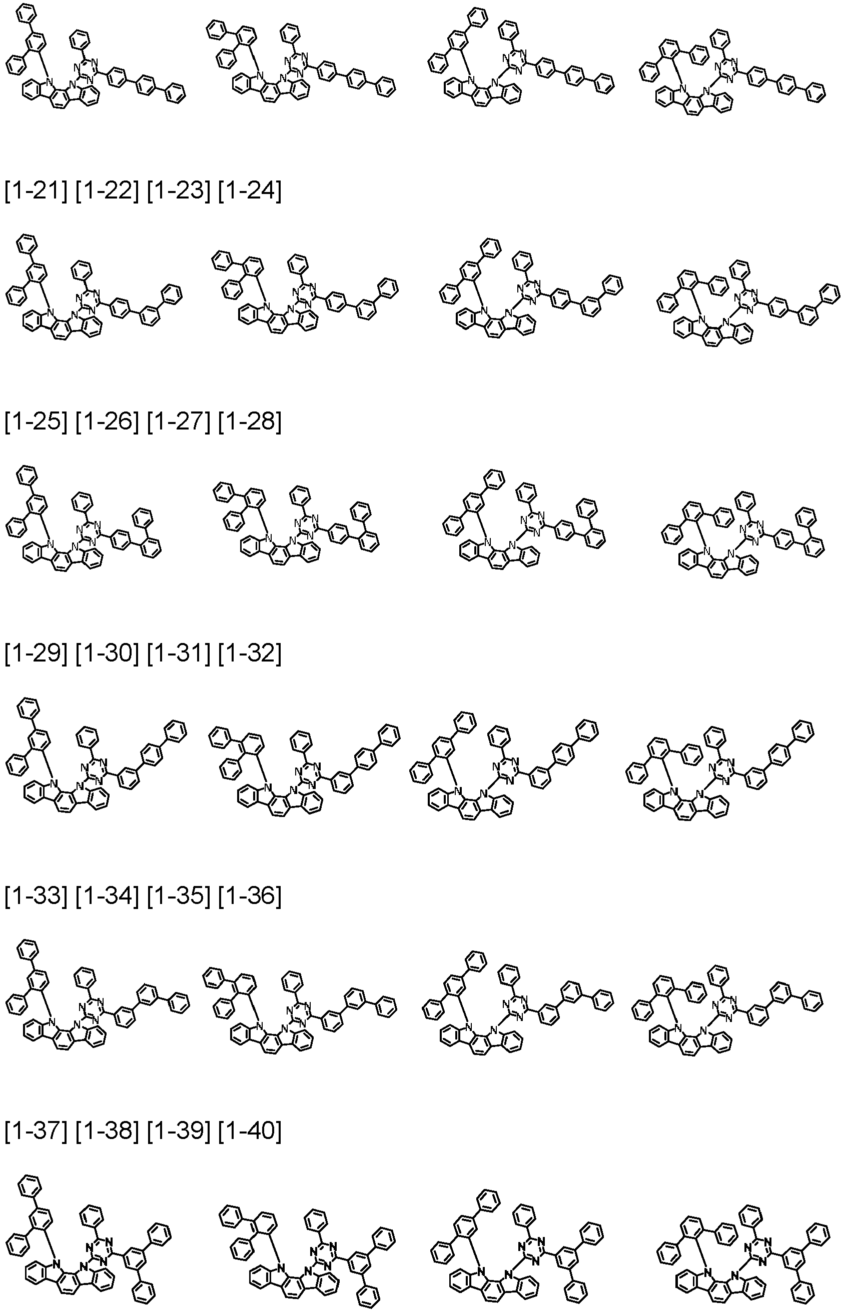

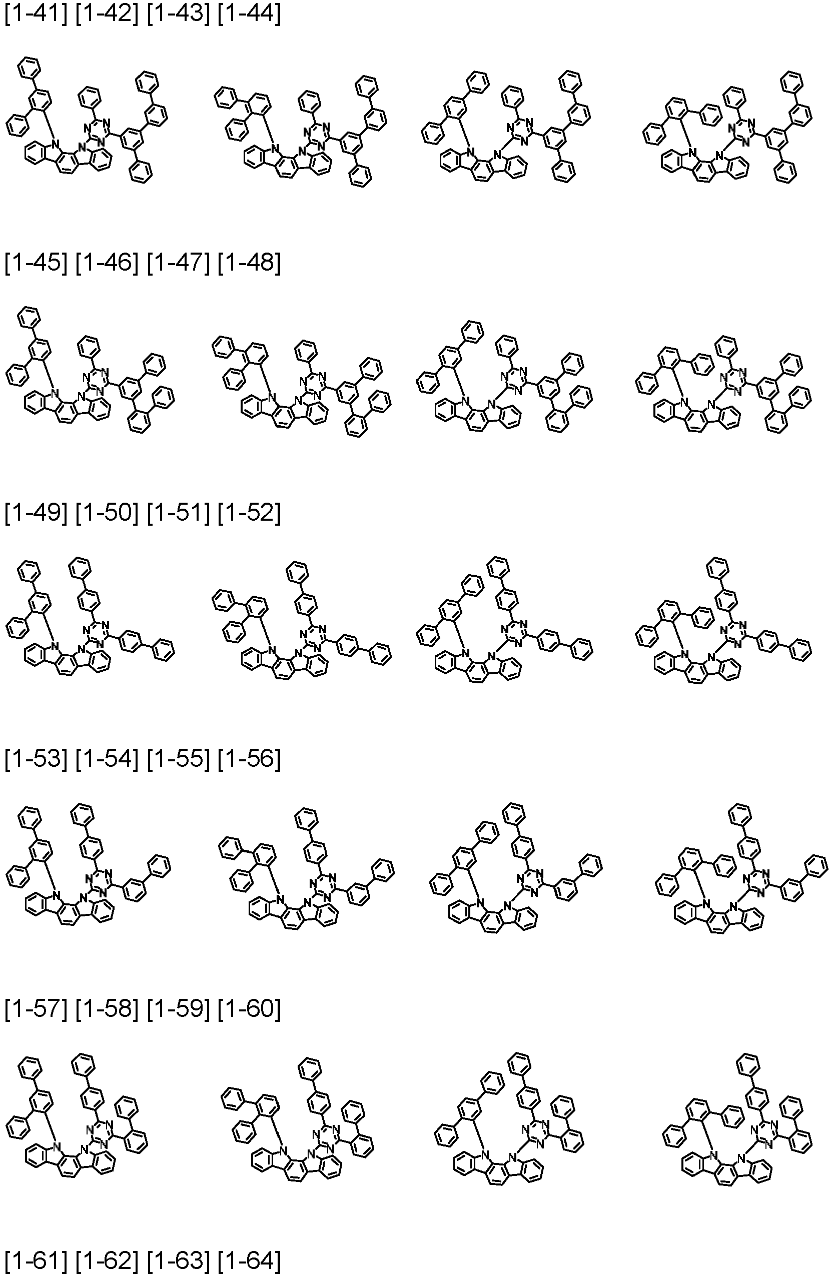

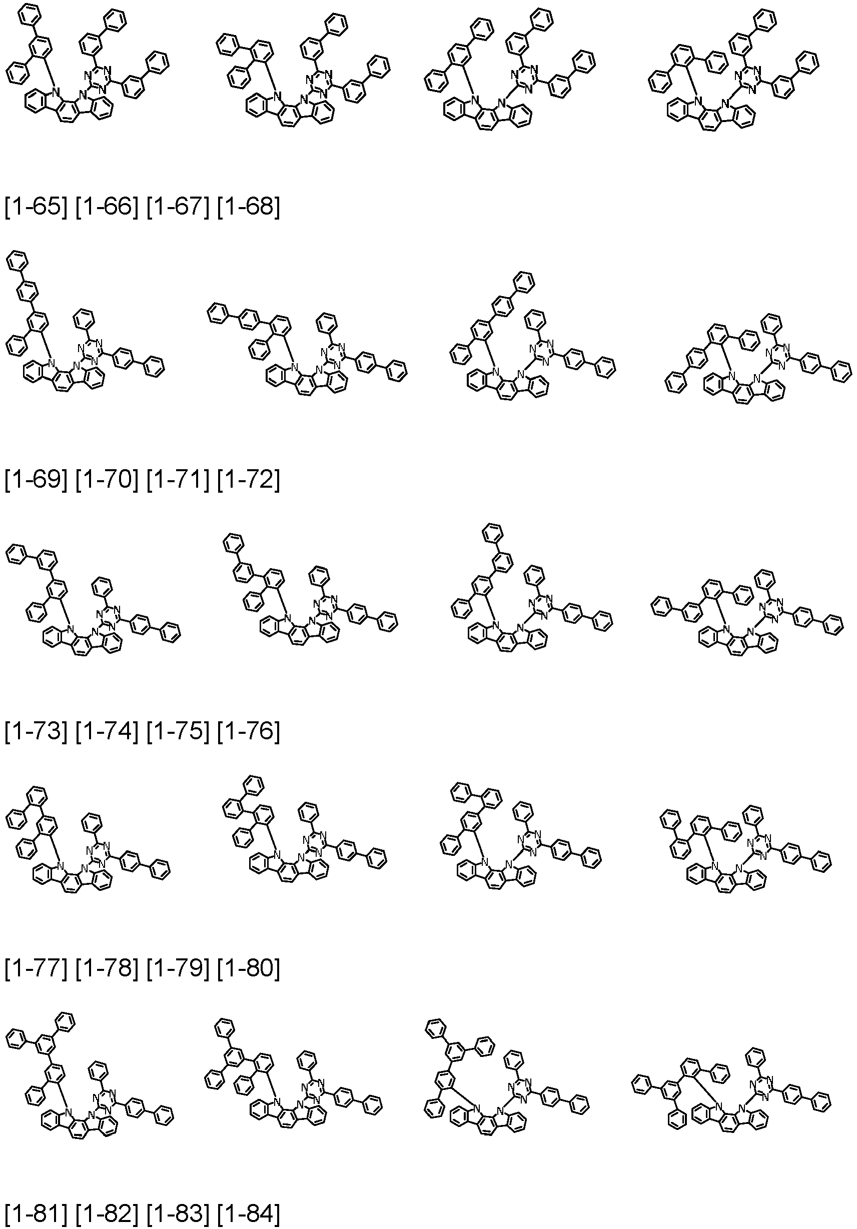

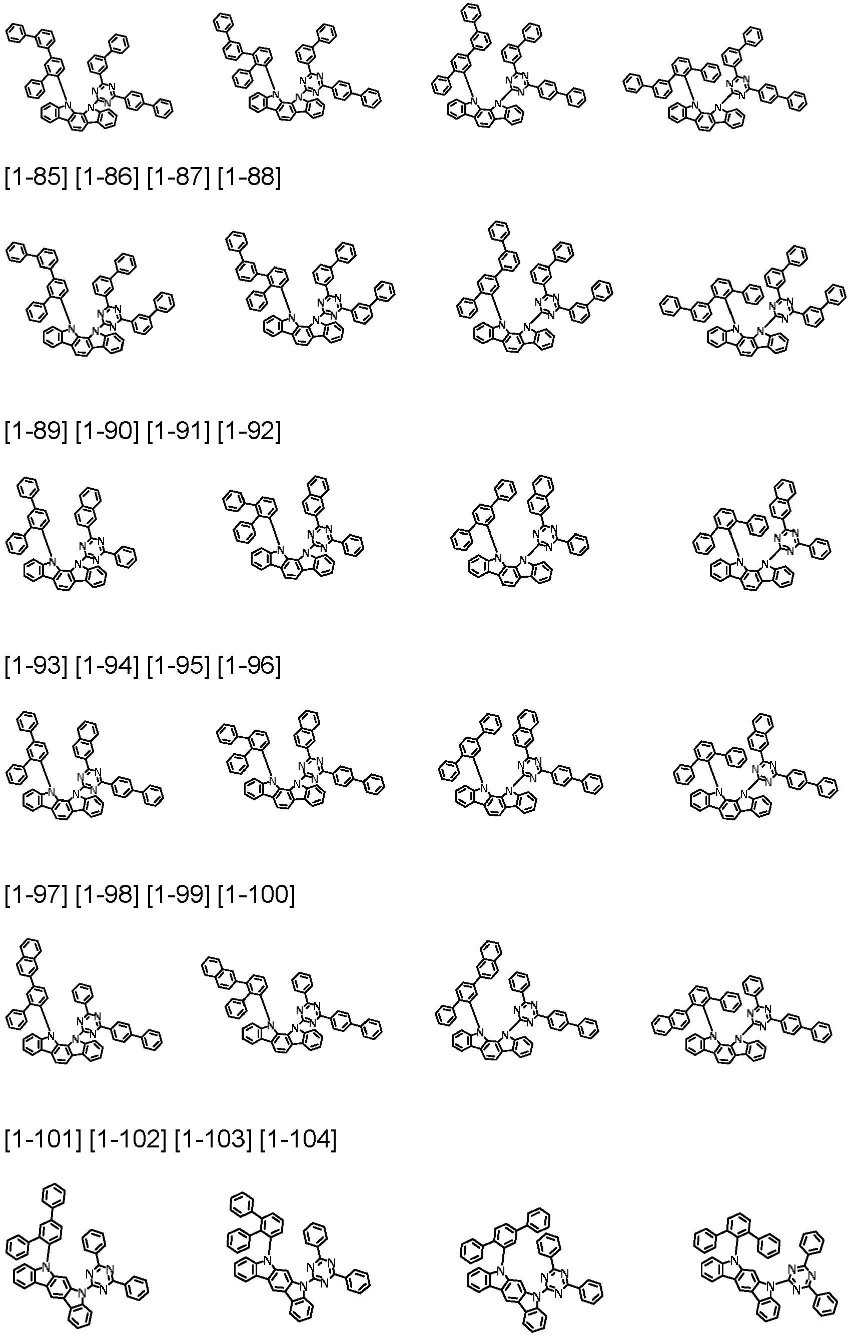

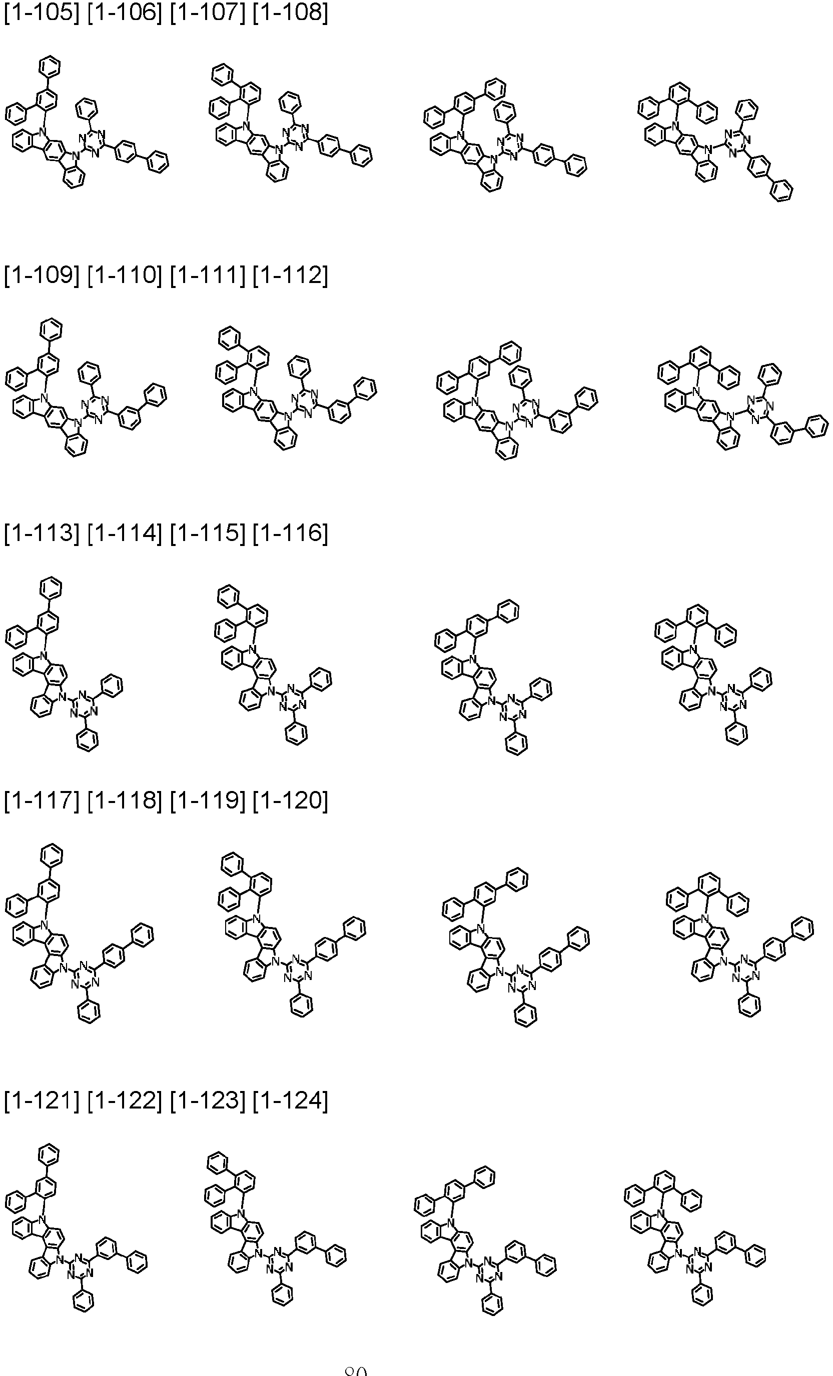

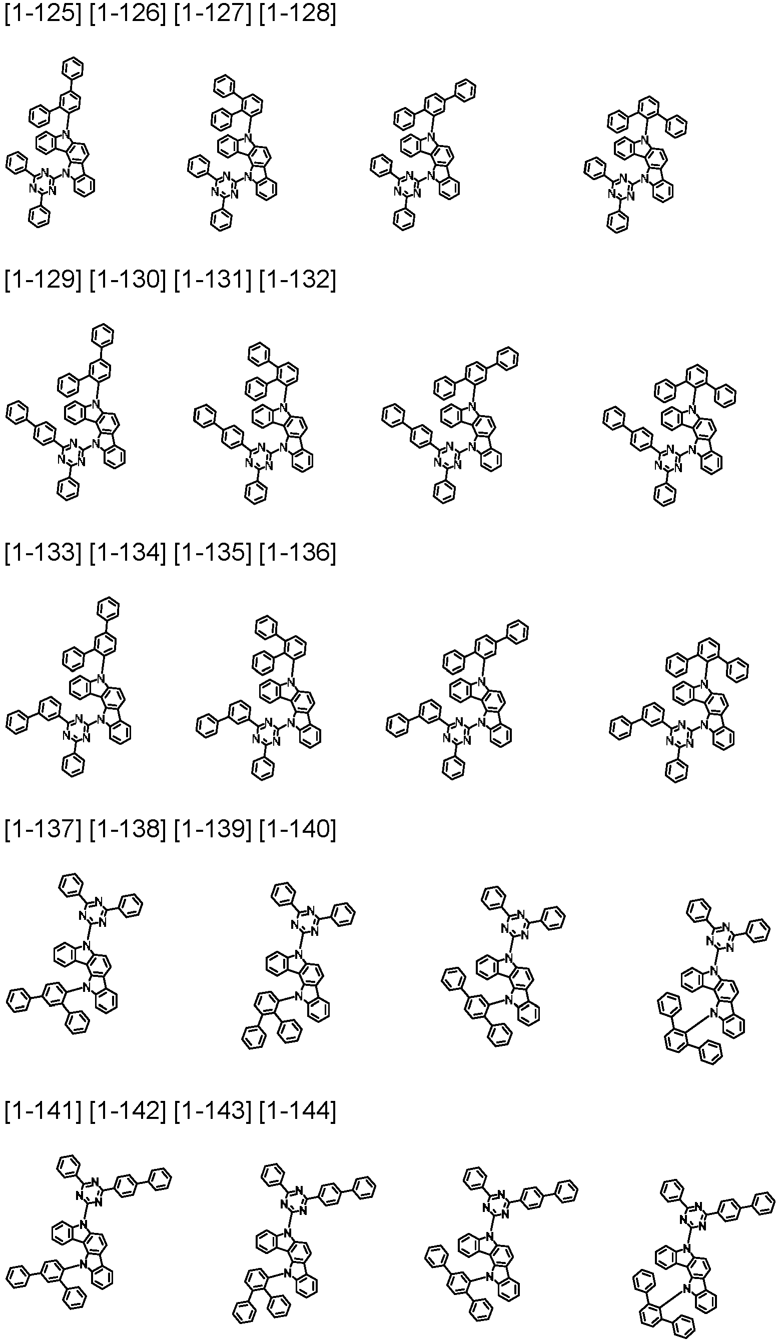

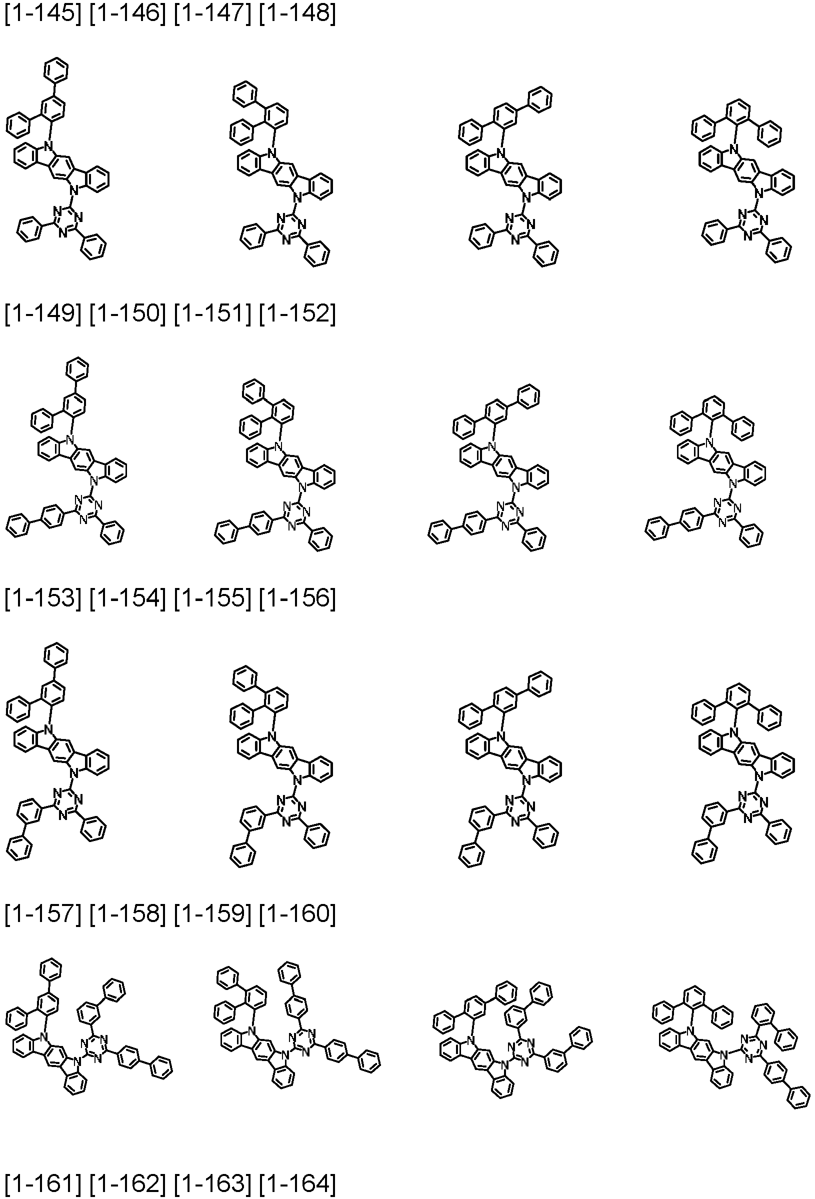

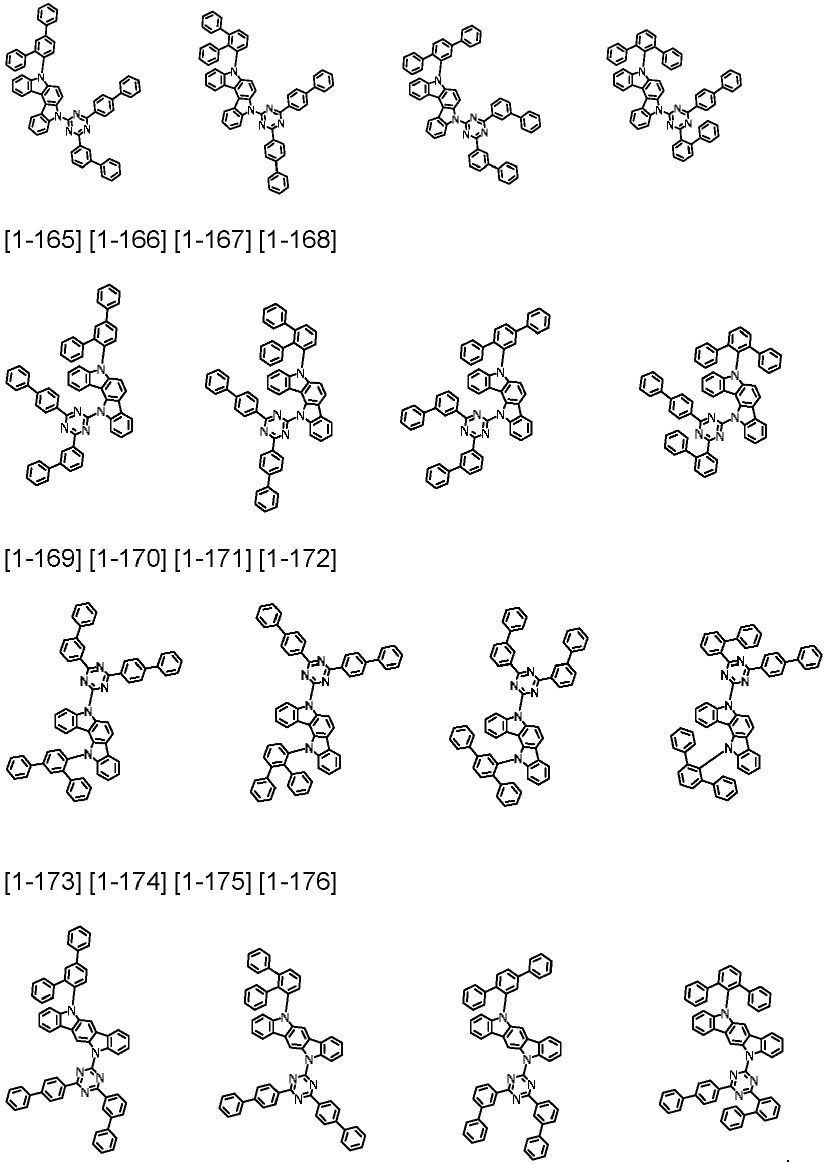

8. A composition for an organic optoelectronic device, comprising
a first compound and a second compound,
wherein the first compound is the compound for an organic optoelectronic device of claim 1, and
the second compound is a compound for an organic optoelectronic device represented by Chemical Formula 3; or a compound for an organic optoelectronic device represented by a combination of Chemical Formula 4 and Chemical Formula 5: 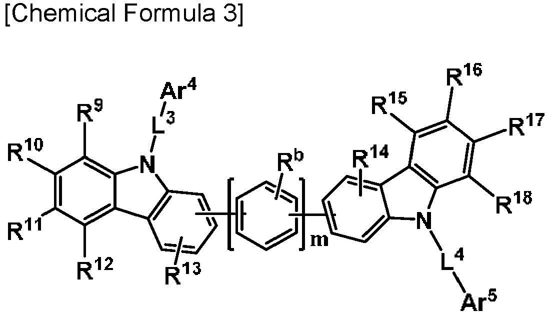
wherein, in Chemical Formula 3,
Ar⁴ and Ar⁵ are each independently a substituted or unsubstituted C6 to C20 aryl group or a substituted or unsubstituted C2 to C30 heterocyclic group,
L³ and L⁴ are each independently a single bond or a substituted or unsubstituted C6 to C20 arylene group,
R^{b} and R⁹ to R¹⁸ are each independently hydrogen, deuterium, a cyano group, a halogen, a substituted or unsubstituted amino group, a substituted or unsubstituted C1 to C30 alkyl group, a substituted or unsubstituted C6 to C30 aryl group, or a substituted or unsubstituted C2 to C30 heterocyclic group, and
m is an integer of 0 to 2; 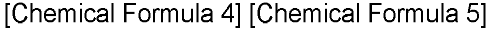 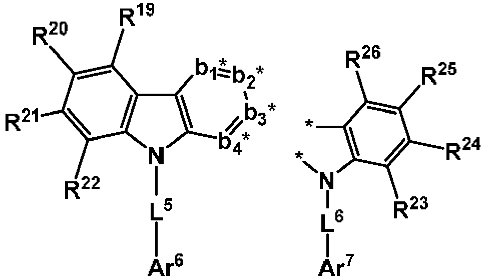
wherein, in Chemical Formulas 4 and 5,
Ar⁶ and Ar⁷ are each independently a substituted or unsubstituted C6 to C20 aryl group or a substituted or unsubstituted C2 to C30 heterocyclic group,
adjacent two of b₁* to b₄* of Chemical Formula 4 are each independently linking carbon linked to * in Chemical Formula 5,
the rest two of b₁* to b₄* of Chemical Formula 4 not linked to * in Chemical Formula 5 are each independently C-L^{b}-R^{c},
L^{b}, L⁵, and L⁶ are each independently a single bond or a substituted or unsubstituted C6 to C20 arylene group, and
R^{c} and R¹⁹ to R²⁶ are each independently hydrogen, deuterium, a cyano group, a halogen, a substituted or unsubstituted amino group, a substituted or unsubstituted C1 to C30 alkyl group, a substituted or unsubstituted C6 to C30 aryl group, or a substituted or unsubstituted C2 to C30 heterocyclic group.

9. The composition of claim 8, wherein Chemical Formula 3 is represented by Chemical Formula 3-8: 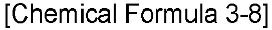 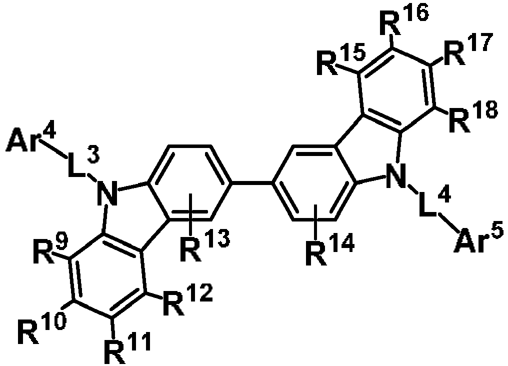 wherein, in Chemical Formula 3-8,
R⁹ to R¹⁸ are each independently hydrogen, or a substituted or unsubstituted C6 to C12 aryl group, and
-L³-Ar⁴ and -L⁴-Ar⁵ are each independently one of substituents of Group II, 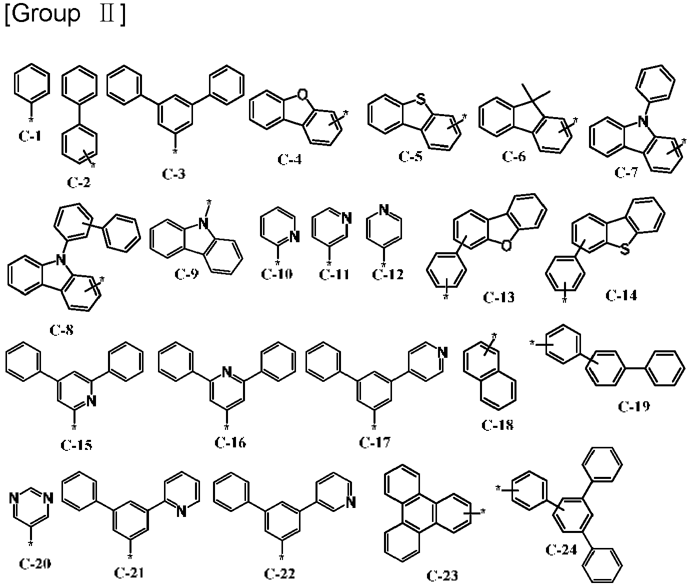
wherein, in Group II, * is a linking point.

10. The composition of claim 8, wherein the combination of Chemical Formula 4 and Chemical Formula 5 is represented by Chemical Formula 4C: 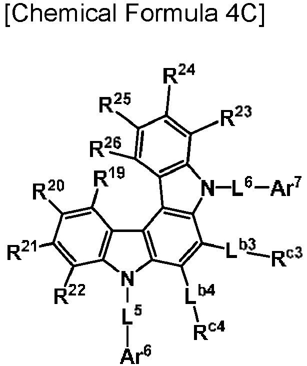 wherein, in Chemical Formula 4C,
L^{b3} and L^{b4} are a single bond,
L⁵ and L⁶ are each independently a single bond or a substituted or unsubstituted C6 to C12 arylene group,
R¹⁹ to R²⁶, R^{c3}, and R^{c4} are each independently hydrogen or a C6 to C12 aryl group, and
Ar⁶ and Ar⁷ are each independently a substituted or unsubstituted phenyl group, a substituted or unsubstituted naphthyl group or a substituted or unsubstituted biphenyl group.

11. An organic optoelectronic device, comprising
an anode and a cathode facing each other,
at least one organic layer between the anode and the cathode,
wherein the at least one organic layer comprises the compound for an organic optoelectronic device of any one of claim 1 to claim 7; or the composition for an organic optoelectronic device of any one of claim 8 to claim 10.

12. The organic optoelectronic device of claim 11, wherein
the at least one organic layer comprises a light emitting layer, and
the light emitting layer comprises the compound for an organic optoelectronic device or the composition for an organic optoelectronic device.

13. A display device comprising the organic optoelectronic device of claim 11.
